# EUROPEAN PATENT SPECIFICATION

(11) **EP 4 457 026 B1**
(45) Date of publication and mention of the grant of the patent: **05.11.2025**
(21) Application number: 22844226.5
(22) Date of filing: 29.12.2022
(51) Int. Cl.: B01L 3/00, G01N 15/14, G02B 21/36, G06T 7/00, G06V 20/69, G01N 33/483, G01N 15/10

(54) **MEANS AND METHODS FOR MODULATING CELLULAR AVIDITY WITH RECEPTORS**
MITTEL UND VERFAHREN ZUR MODULATION DER ZELLAVIDITÄT MIT REZEPTOREN
MOYENS ET PROCÉDÉS DE MODULATION D'AVIDITÉ CELLULAIRE AVEC DES RÉCEPTEURS

(30) Priority: 31.12.2021 NL 2030387; 13.05.2022 EP 22173281
(43) Date of publication of application: 06.11.2024
(73) Proprietor: LUMICKS CA Holding B.V., 1105 AG Amsterdam (NL)
(72) Inventor: DAVOLI, Serena Alba, 1105 AG AMSTERDAM (NL); YU, Zhongjie, 1105 AG AMSTERDAM (NL); RIJO DA COSTA CARVALHO, Rui Pedro, 1105 AG AMSTERDAM (NL); GREGG, Trillian Ashley, 1105 AG AMSTERDAM (NL); VAN LOENHOUT, Marinus Theodorus Johannes, 1105 AG AMSTERDAM (NL); DE GROOT, Mattijs, 1105 AG AMSTERDAM (NL)
(74) Representative: Algemeen Octrooi- en Merkenbureau B.V.
(86) International application number: PCT/EP2022/088001
(87) International publication number: WO 2023/126473

(56) References cited:
- WO-A1-2021/089654
- US-A1- 2021 364 439

## Description

### Introduction

It is understood in the art that immune receptors e.g. TCR, CAR with the highest affinity may not necessarily give optimal immune responses *in vitro* and *in vivo* to a target cell, *e.g.* a cancer cell. Direct correlations between receptor affinity and *in vivo* performance are often absent. Cellular avidity, the overall strength of interactions between a diversity of receptor-ligand pairs at the cell surface, is a better predictor for immune cell functionality and optimizing cellular avidity to specific ranges may also allow to optimize *in vivo* performance.

This means that for a productive and durable immune response a transgenic TCR-T cell, or the like such as with a CAR-T cell, can be understood to require sufficient cellular avidity to kill the target cells, while having a cellular avidity which is not too high to avoid *e.g*. exhaustion or perhaps unwanted side effects such as on-target/off-tumour toxicity. Hence, there is an interest in the field to select TCRs, or the like, which differentiate with regard to cellular avidity.

In the art, identifying or selecting receptors with advantageous and/or different cellular avidities is a highly cumbersome and time consuming process. A prior art method for measuring cellular avidity is known from patent application WO 2021/089654 A1. Current methods involve many different screening methods wherein after laborious selection methods clones are identified that still need *in vivo* validation as the selection assays only partially correlate function. Currently, cellular avidity is not determined in screening procedures because the few tools that there around are very laborious, low throughput, and/or require expert tools and handling. For many applications, and in particular for personalized medicine, it would be highly advantageous if the process for characterizing T cells, CAR-T cells, TCRs, and the like, and their properties including the cellular avidity, would be highly efficient. Moreover, it would also be of interest to provide means and methods with which cellular avidity may be controlled.

Hence, the current inventors now sought to provide i.a. for improved means and methods for characterizing TCRs of interest, for modulating their cellular avidity as well.

### Summary of the invention

The current inventors working with a particular CAR-T population now surprisingly discovered that the expression level of a CAR receptor has an effect on cellular avidity. This means that cellular avidity is not necessarily an inherent property of the receptor itself, but also depends on the amount of receptors present on the cell membrane. Advantageously, as the inventors in applying means and methods involving cellular avidity, made this discovery, this means that this knowledge can now be applied in means and methods utilizing cellular avidity.

For example, for any candidate receptor, controlling expression level may allow controlling cellular avidity. Hence, when candidate receptors are identified, it may be opted to prepare and select gene construct that provide for different expression levels and provide for different cellular avidity. As cellular avidity is believed to be relevant for function, this may be an important aspect part of clinical development. As another example, as it was also shown in the examples provided herein that cellular avidity methods can enrich cells with a higher or lower receptor expression, this may be of use in a scenario wherein such enrichment could be of benefit. For example, in a scenario wherein cells with a high expression level, *i.e.* high copy number, would be undesirable, applying a cellular avidity method (*e.g*. shear force), may enrich for the low copy number cells. This way, utilizing a relatively mild process step, advantageous enriched cell fractions may be obtained which are of use, e.g. for clinical applications wherein high copy number cells may be more associated with risks.

### Figures

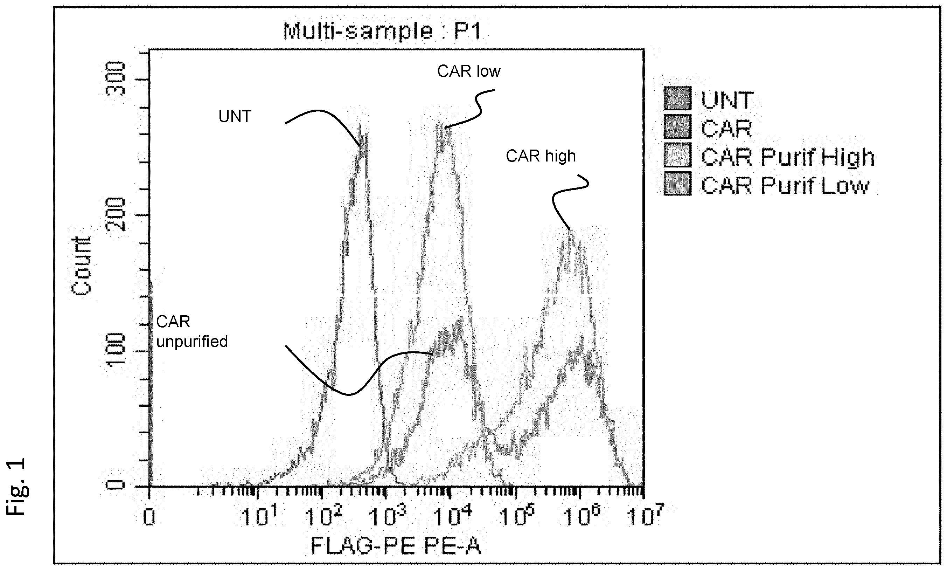

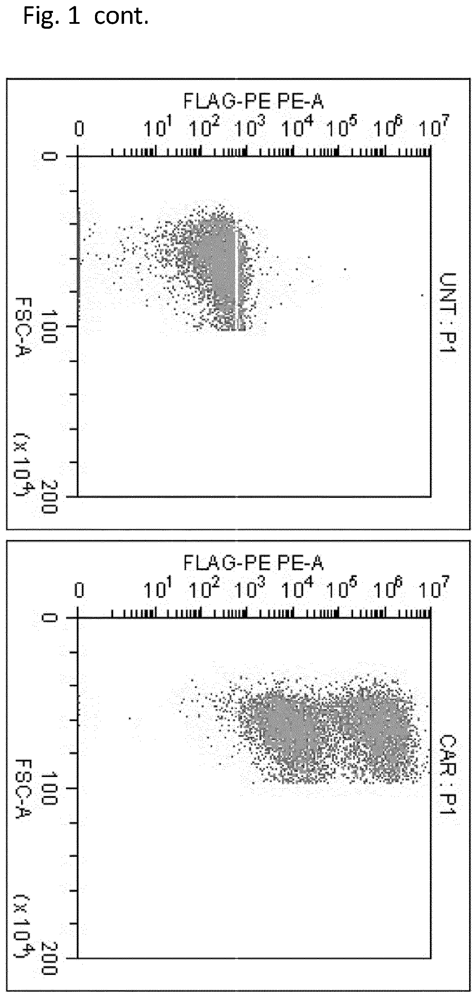

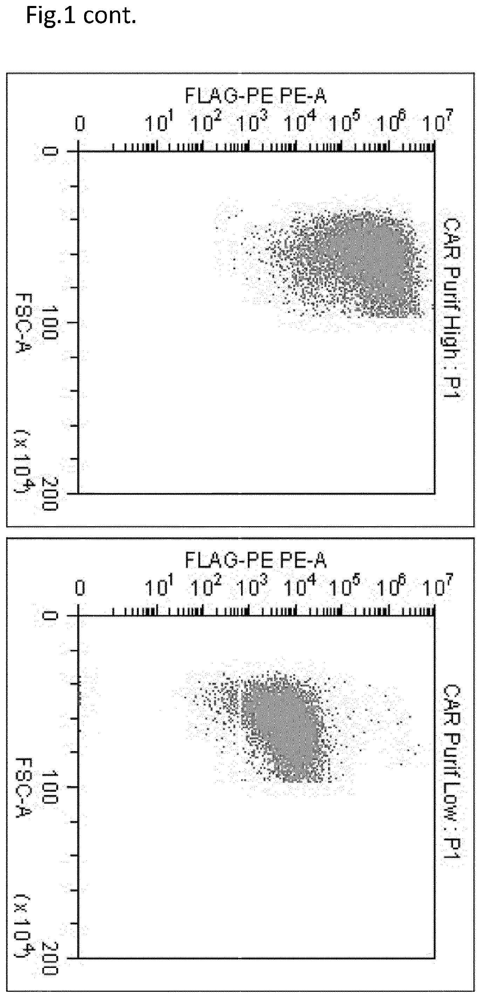

Figure 1. FACS analysis of untransduced and CAR transduced primary T cells separated for CAR expression levels using MACS. In the CAR fraction two populations can be identified, identified as CAR low and CAR high. Untransduced cells have lower staining as compared with CAR low.

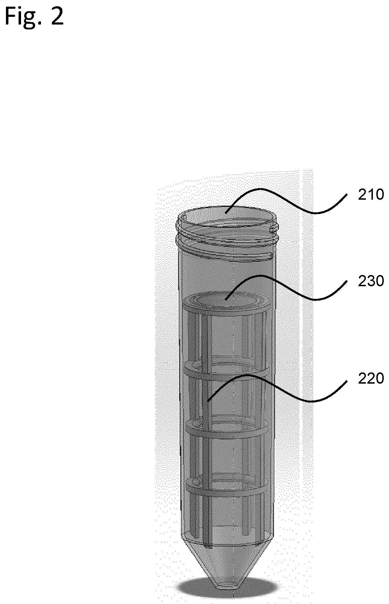

Figure 2. Schematic depiction of a 50 ml centrifuge tube (210), 3D-printed slide holder (220) and 18 mm glass slide (230) used in centrifuge experiments.

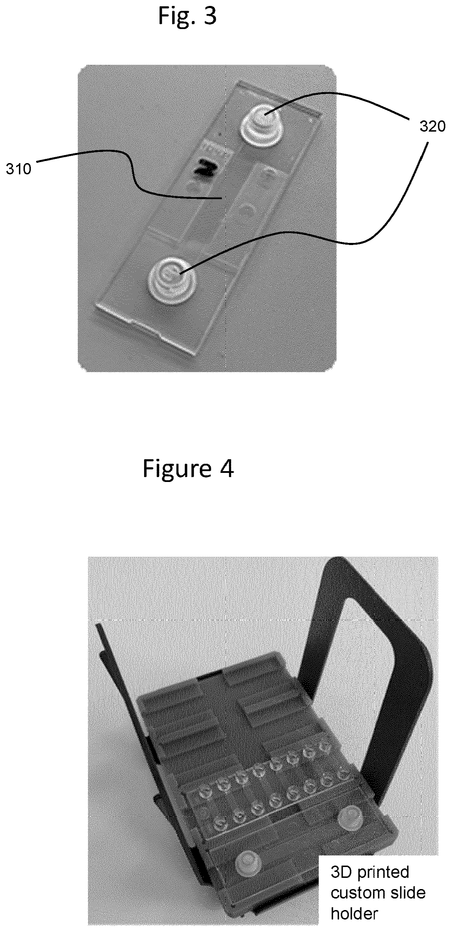

Figure 3. Photograph of a Ibidi channel slide used in centrifuge experiments. Indicated are the position of the main channel (310) and the channel inlet and outlet (320).
Figure 4. Photograph of a 3D printed slide holder that fits into the bucket of a centrifuge for holding Ibidi channel slides.

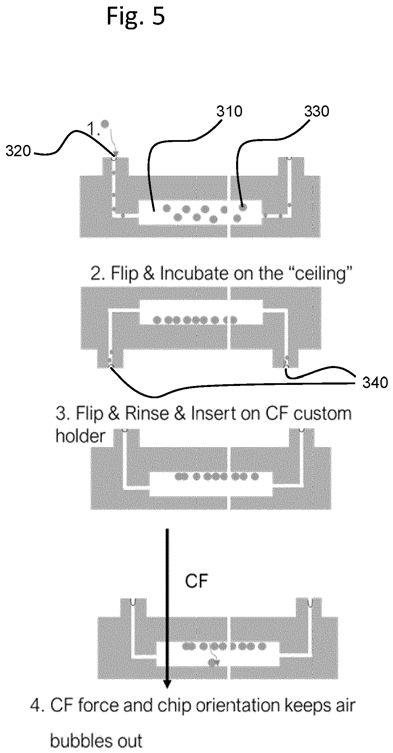

Figure 5. Schematic describing the procedure for performing centrifuge experiments using *e.g*. Ibidi channel slides. Showing in step 1 the loading of cells (330) into the channel (310) via channel inlet (320). In step 2 the channel slide is flipped upside down to allow the cells (330) to sediment to the "ceiling" of the channel (which is optionally coated with target cells as described herein). Some of the cells sediment into the channel inlet and outlet but the cells and fluid are trapped inside the channel slide due to capillary force as indicated by the fluid meniscus (340). In step 3 the channel slide is flipped upright with the cells now "hanging" from the ceiling (the top side of the fluid channel). In step 4 a centrifugal force is applied indicated with the black arrow labelled CF and cells may detach from the target cells and/or channel wall as indicated here for a single cell.

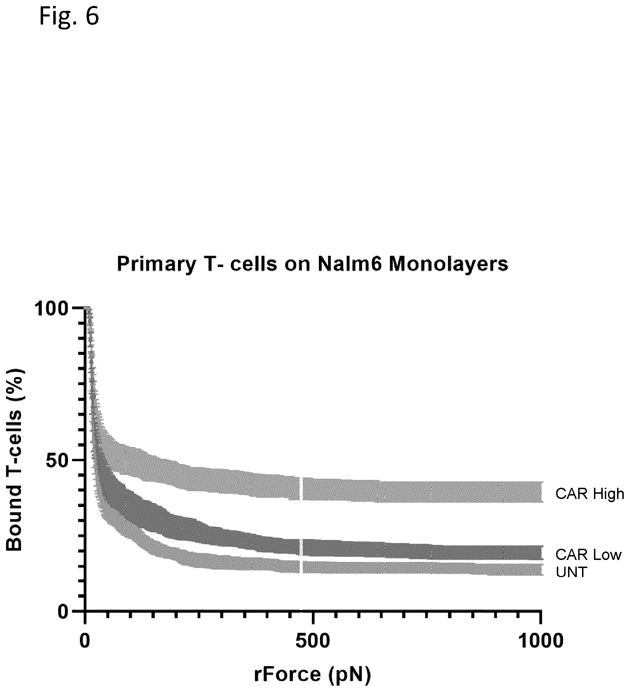

Figure 6. Cellular avidity plot showing different fractions analysed separately in a z-Movi^{®} device. The graph plots the percentage of bound cells as a function of the applied acoustic force. The grey bands show for each cell type (UNT, CAR Low, CAR High) the average +/- SEM (standard error of the mean) from 4 independent runs (as described below).

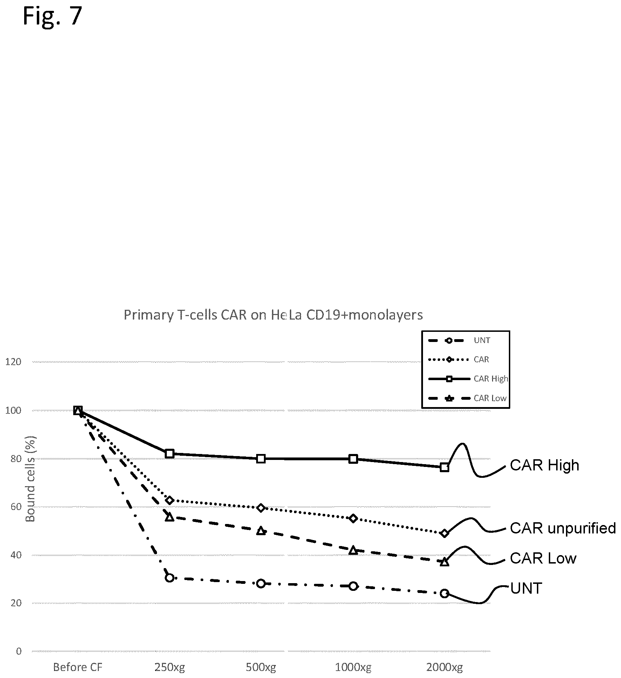

Figure 7. Cellular avidity plots obtained using a 18 mm slide centrifugal force experiment. The graph shows the percentage of bound cells as a function of the applied centrifugal force (expressed in g). Shown is that four populations of primary T cells with different CAR expression levels (CAR High and CAR Low), or no CAR expression (UNT), can be distinguished and separated/enriched using centrifugal force.

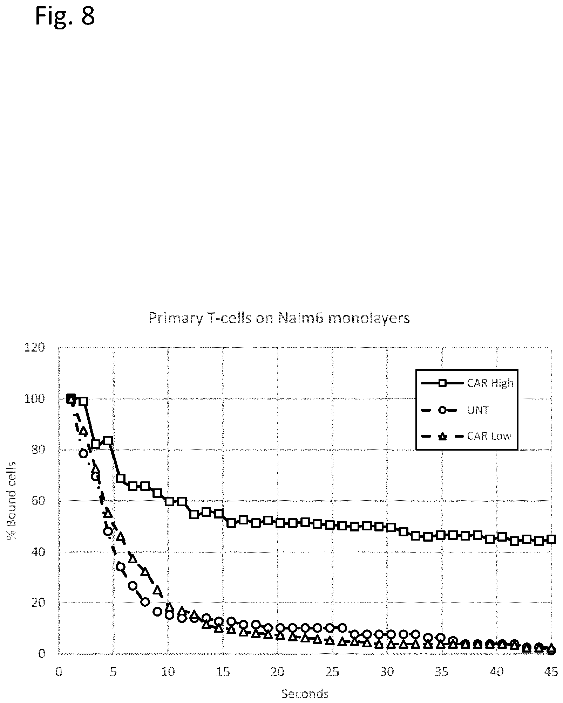

Figure 8. Cellular avidity plots obtained in a shear-flow experiment. The graph plots the percentage of cells bound as a function of time. In the experiment time correlates with an applied shear stress (see description below). Shown is that primary T cells with different CAR expression levels (UNT, CAR Low and CAR High) can be distinguished and separated/enriched using shear force.

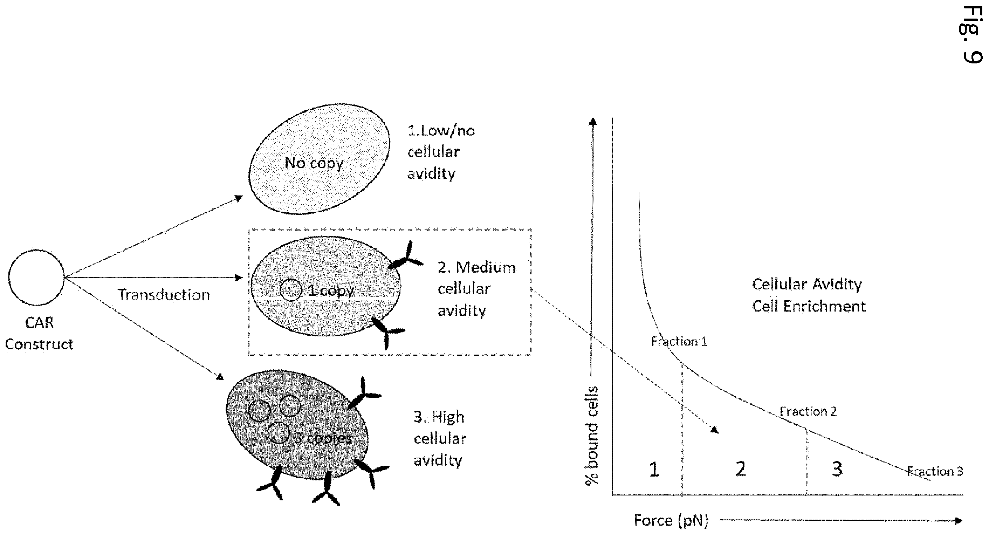

Figure 9. Schematic outlining preparing fractions from a mixture of cells having no copy of a vector genome, 1 copy and 2 or more copies. In the scheme outline, by collecting fraction 2, a fraction highly enriched for cells with medium expression levels, e.g. representing 1 vector copy per cell, can be obtained.

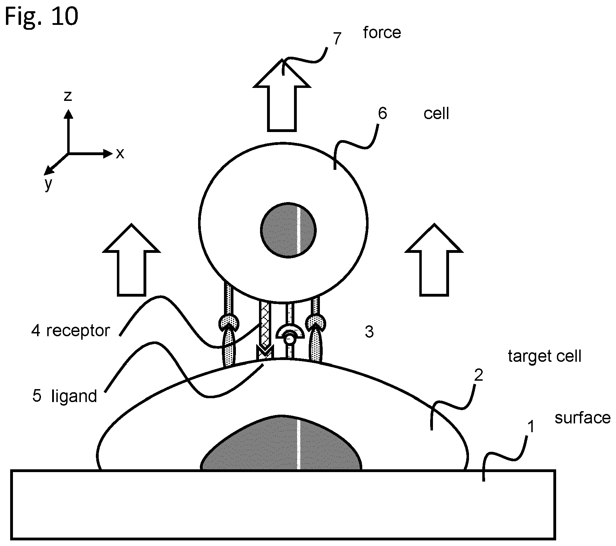

Figure 10. Schematic showing target cells and cells of interest and cellular avidity. Target cells (2) are provided on a surface (1), which as depicted is in this case a flat surface. The target cell expresses ligands and receptors, likewise the cell to be targeting (6) the target cell expresses ligands and receptors as well. A specific ligand-receptor interaction (4 and 5) can be the driving force for the forming of a cell-cell bond with multiple ligand-receptor interactions combined resulting in strong cell-cell binding (3). To rupture this cell-cell bond, a force (7) is exerted on the cell (6) away from the target cell (2), which can be in the z-axis direction e.g. when the flat surface is defined as being in the x-y plane. Alternatively, this can also be in the x- or y-axis direction. When the cell-cell bond is ruptured, the cell moves away from the cell surface and / or the target cell and this event can be detected and/or detached cells can be collected and quantified and/or further analyzed.

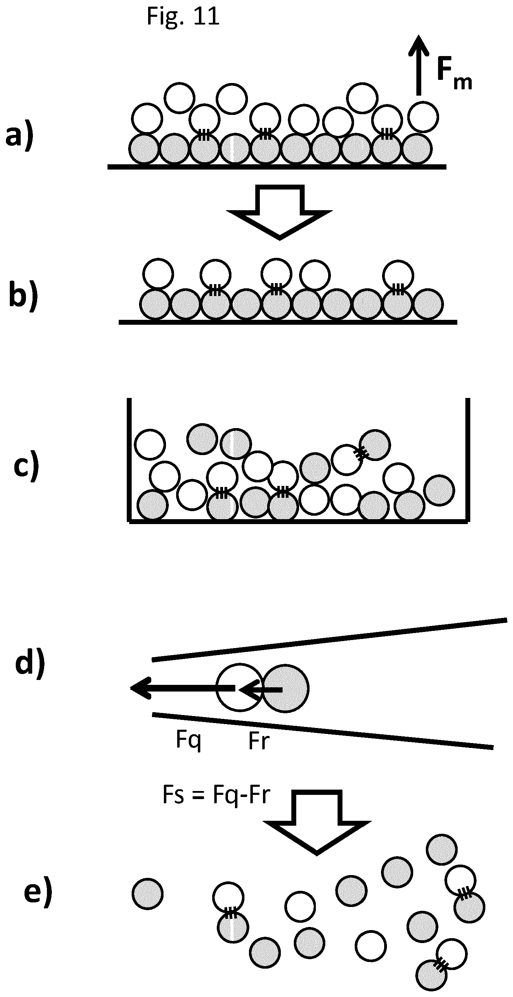

Figure 11. In a cellular avidity method utilizing applied forces involving cells attached to a surface (depicted as grey cells), cells (depicted as white cells) are interacted with the attached cells to bind therewith, e.g. utilizing target cells expressing an antigen and cells of interest such as cells expressing a receptor, e.g. an effector cell with a CAR against the antigen, as depicted in a). After a defined incubation, a force, Fₘ applied away from the attached cells. This results in cells moving away therefrom, either because they did not bind to the cells attached to the surface or because the binding strength was not strong enough, *e.g*. when aspecifically bound. Cells that remain bound were sufficiently strongly bound to the attached cells as depicted in b). The binding of cells with a receptor in accordance with the invention can depend on expression level. Such scenarios are *e.g*. employed in the examples as described herein. Cells that formed a strong binding, *e.g*. a synapse, may substantially remain (indicated with hashes). Alternatively, a cellular avidity method can be performed which does not require cells attached to a surface. In such a scenario, e.g. target cells (depicted as white cells) are provided and cells with different levels of expression of a receptor, *e.g*. effector cells (depicted as grey cells), and these are incubated for a defined period. After said incubation, cells are resuspended and a differential force, Fₙ, is applied. Such an applied force may be larger than typically used when cells are attached (Fₘ). This force may break cell-cell bonds, preferably aspecific cell-cell or bonds with less strong binding. For example, aspecific cell-cell bonds may be less strong when compared with specific cell-cell bonds involving e.g. synapse bonds, thereby resulting *e.g*. substantially specific or strong cell-cell bonds. The cell suspension may be subsequently analysed with regard to singlets (either white or grey cells) and doublets (grey cell bound with white cell), which numbers may be used, *e.g*. to provide a cellular avidity score. One may also apply a selected force which allows to differentiate between different expression levels of receptors in order to enrich for cells with regard to higher or lower expression level of the receptor or higher or lower copy number or of a defined copy number, *e.g*. in a scenario wherein a CAR receptor or the like is provided *e.g*. to effector cells. Of course, one may also combine applying a differential force (shown in d), after a force has been applied, e.g. away from attached cells (in the scenario obtained as shown in b), or one may apply a differential force after incubation on attached cells (in the scenario as obtained in a)). Cellular avidity scores may be determined by determining the number of grey cells and white cells bound to each other (doublets) and/or by counting the number of grey cells bound to white cells, e.g. effector cells bound to target cells, that formed a specific bond such as a synapse (doublets with hashes). This way, *e.g*. defined expression levels in particular associated with *e.g*. synapse formation may be identified. Cellular avidity scores can be calculated taking into account initial amount of cells provided and/or single cells obtained in the methods. For example, by calculating the ratio of the number of cells of interest that remained bound to target cells after exerting the force to the number of cells of interest initially provided.

### Detailed description

As said, the current inventors working with a particular CAR-T population surprisingly discovered that the expression level of a CAR receptor has an effect on cellular avidity. This means that cellular avidity is not necessarily only an inherent property of the receptor itself, but may depend on the amount of receptors present on the cell membrane as well. Advantageously, this aspect can be of use in means and methods utilizing cellular avidity in accordance with the invention as described herein.

It is understood that "cellular avidity" as used throughout herein comprises the overall strength of interactions occurring in a cell to cell contact, involving a diversity of molecules at the surfaces of the cells that interact (see *e.g*. Figure 10). Such interactions may include a diversity of receptor-ligand pairs, among which e.g. a specific receptor-ligand interaction, occurring at the membrane surface of a cell. For example, when a T cell receptor triggers the formation of an immune synapse by recognizing an antigen presented by an MHC molecule at an antigen presenting cell, the synapse formation involves such multitude of interactions, as also other membrane bound molecules are involved in the interactions (such as integrins and the like). Hence, "cellular avidity" may not be restricted to the interaction of *e.g*. the alpha and beta chain of the TCR and the antigen presented by MHC, but rather involves a multitude of interactions working jointly forming a strong bond between *e.g*. cells. It may also involve active signalling and processes internal to the cells such as *e.g*. during immune synapse formation. It is understood that the cellular avidity of a cell of a certain type is defined relative to its target cell and conditions tested.

The term ligand and receptor in accordance with the invention may define their inter-relationship. The term receptor may not be construed to be limiting in any way and is understood to mean a protein presented at the cell surface which can (specifically) interact with another protein (ligand) presented at a another cell. The terms ligand and receptor are used to indicate a complementarity which is important for specific recognition between cells without restrictions on the complementary molecules that can be contemplated.

In a first embodiment, a method is provided for differentiating between cells having different expression levels of a receptor by determining cellular avidity, comprising the steps of:
a) providing cells with different expression levels of a receptor;
b) providing target cells attached to a surface;
c) contacting each of the cells with different expression levels of the receptor with the target cells attached to a surface to allow the cells to interact with the target cells;
d) exerting a force on the cells with different expression levels of the receptor;
e) determining of each of the cells with different expression levels of the receptor the number of cells that detached from and/or remained attached to the target cells;
f) assigning cellular avidity scores to each of the cells with different expression levels.

In step a) of the method, cells with different expression levels of a receptor are provided and in step b) target cells attached to a surface are provided. The target cells in accordance with the invention are defined to be the cells immobilized, *i.e.* attached to a surface. The cells with different expression levels of a receptor may preferably not be immobilized. It is understood that in accordance with the invention target cells and a cell expressing a receptor relate to two different cells which are to interact specifically with each other. Which cell is immobilized may not be of importance for determining cellular avidity between these cells. As long as a force can be applied to the cells binding to the immobilized cells, and detachment and/or attachment of cells can be determined, determination of the cell numbers thereof allows one to determine cellular avidity. Of particular interest and as further described below, in accordance with the invention, cells of interest can be T cells or the like, which are to specifically target e.g. a cancer cell or other antigen presenting cell presenting a defined antigen on its surface. Hence, accordingly, in methods of the invention the target cells attached to the surface, *i.e.* the immobilized cells, may be cancer cells, and the cells with different expression levels of receptors provided may be T cells. This may be in particular advantageous if in a cell population different expression levels are combined, e.g. for enrichment purposes. Conversely, in methods of the invention, one may also choose to have the T cells immobilized, as a target cell, and have the cancer cell as a cell of interest. The latter may complicate enrichment of T cells, but may be advantageous in scenarios where certain cancer cells are difficult to immobilize and/or different cancers cells may be studied.

With regard to the cells having different expression levels of a receptor, it is understood that this means that the cells are to express the same amino acid sequence at its cell surface where it is to interact with the target cells. There are means and methods known in the art with which expression levels of proteins and thus receptors can be well controlled. For example, promoter sequences driving expression of the receptors may be varied, resulting in different expression levels. Alternatively, 5'-UTR, 3'UTR, splice signals and/or codon usage at the RNA level may also affect RNA stability and translation and thus variation therein may allow for different expression levels. Furthermore, other transcription and/or translation factors may have an effect on expression levels. Different inducible promoters may be utilized to vary expression level as well. In any case, different constructs can be made providing for different expression levels of the same receptor sequence, or cells can be genetically modified to achieve different expression levels of the same receptor sequence. Different copy numbers of the same expression cassette can also be used as a means to provide for different expression levels. It is understood that with regard to receptor, this includes at least e.g. a CAR-T receptor or a TCR receptor, the latter of which is composed of an alpha and beta chain. In any case, of each cell with a different expression level, a plurality is to be provided, *i.e.* a number of cells that allows to determine a cellular avidity score in accordance with the invention as described herein. Hence, in one embodiment, the cells with different expression levels of the receptor are provided by providing cells with different expression constructs expressing the same receptor. In another embodiment, the cells with different expression levels of the receptor are provided by providing cells with different copy numbers of an expression construct expressing the same receptor. In yet another embodiment, in accordance with the invention, the cells with different expression levels of the receptor are provided by providing cells with different expression constructs expressing the same receptor and providing cells with different copy numbers of an expression construct expressing the same receptor.

The target cells are provided attached on a surface. Providing the target cells attached to a surface is well known in the art. For example, a glass or plastic surface may be utilized to attach cells thereto. A surface material may be preferred which allows for detection of attachment to and/or detachment from the target cells, *i.e.* cells carrying a receptor. Such a surface material for instance also may allow for microscopy methods (e.g. by being a transparent material). In order to attach cells to the surface, the surface may be pre-treated with a coating such as a polypeptide. Suitable polypeptides include e.g. poly-L-lysine or the like. Suitable polypeptides for attachment of target cells that may be contemplated and are known in the art include for example fibronectin, poly-L-lysine, poly-D-lysine, poly-L-ornithine, laminin, collagen, fibronectin, fibrinogen, vitronectin, or osteopontin. In any case, a suitable polypeptide or other suitable coating if needed may be selected such that the target cells that are attached to the surface allow for the target cells to remain attached to the surface when applying a force on the cells carrying the receptor. In other words, when a force is applied on cells of interest which allows for these cells to detach from the target cells, the target cells are to substantially remain attached to the surface.

In another embodiment, instead of providing target cells attached on a surface, a functionalized wall can be provided which provides the cells with a receptor a suitable surface to attach to in the means and methods in accordance with the invention as described herein throughout. A functionalized wall in accordance with the invention presents ligands/receptors in a similar fashion as they are presented on a cell surface, e.g. in a lipid bilayer, or the like. A functionalized wall thus preferably is functionally equivalent to target cells attached to a surface, and mimics target cells attached to a surface.

In an alternative embodiment, the cells with the receptors having different expression levels, when provided separately, may be provided attached to different surfaces as different target cells, when, and, subsequently the cells previously used as target cells, *e.g*. a cancer cell (in other embodiments) instead and these cells may be allowed to interact therewith. Cells that have detached and/or remained attached can be subsequently determined and cellular avidity scores provided for each of the cells with different expression levels, and cellular avidity scores can be likewise compared.

In step c) the cells with different expression levels of the receptor are contacted with the target cells attached to a surface to allow the cells to interact with the target cells. Hence, in this step, the cells with different expression levels of the receptor are introduced on the target cells, *e.g*. by layering the cells thereon. It is understood that this step of contacting is well controlled. For example, as shown in the example section, the step of contacting may be a defined period of 5 minutes. Of course, the step of contacting may be shorter or longer, for example in the range of about 2 minutes to about 15 minutes.

Subsequently, in step d) a force is exerted on the cells with different expression levels of the receptor, wherein the force is in a direction away from the target cells. It is understood that in this step, the force applied may be perpendicular (in the direction of z-axis) to the surface (x,y) to which the target cells are attached, for example when a centrifugal force or acoustic force is applied. The force may also be lateral (x-axis or y-axis), for example when a shear force is applied (see *e.g*. Figure 10). In any case, the force is applied and is controlled such that a defined force is exerted on cells carrying the receptor that interacted with the target cells (and formed a bond or not). It is understood that the force that is exerted on the cells attached to the target cells is to be substantially equal for all cells, such can be achieved *e.g*. when using a flat surface as depicted in Figure 10. Other suitable surface shapes may be used (*e.g*. a tube with exerted concentrical force or laminar flow force in the direction of the length of the tube), as long as the force exerted can be substantially equal at a defined surface area, such a surface shape may be contemplated. The force required to move a cell away from the target cell preferably can be detected, *e.g*. via microscopy or other means, to which may be referred to as a cell detachment event. This way, cell detachment events can be monitored and counted.

In step e) the number of each of the cells with different expression levels of the receptor that have detached from and/or remain attached to the target cells attached to the surface in step d) are determined, and using the determined number, in step f) cellular avidity scores are assigned to each of the cells with different expression levels. By knowing the number of cells that have interacted with the target cells, and knowing the number of cells that remain attached to the target cells, the percentage of each of cells with different expression levels of the receptor that remain attached to the target cells can be determined. The percentage of cells from the contacting step c) that remain attached to the target cells can be well determined. This can be done by relatively simple means known to the person skilled in the art, *e.g*. by simply providing a defined number of cells to interact with the target cells and, after incubation and applying the force, subsequently collecting detached cells and determining the amount of cells collected, and calculating the percentage that remains bound therefrom. Of course, it may be advantageous and convenient to use microscopy, with which attached cells can be identified and quantified and detachment can be likewise monitored and quantified. As shown in the examples, the z-Movi^{®} device applying an acoustic force is well equipped to do so. Likewise, similar devices may be provided with microscopy or other means to quantify cells, and attachment and/or detachment events, and also utilizing *e.g*. shear-force or centrifugal forces instead of acoustic force). Anyhow, at least based on determined numbers of each of the cells with different expression levels of the receptor (that have detached and/or remain attached in step d)) a cellular avidity score for the each of cells with different expression levels of the receptor can be determined.

With regard to the cellular avidity score, it is understood that this is to express the strength of binding of cells carrying a receptor to the target cells. It is understood that where we refer to specific forces applied to cells this may refer to average forces, *e.g*. such forces may not be fully homogeneous, for example over the contact surface as may be the case with acoustic forces and shear-flow forces (see *e.g*. Nguyen, A., Brandt, M., Muenker, T. M., & Betz, T. (2021). Lab on a Chip, 21(10), 1929-1947 for a description of force inhomogeneities in acoustic force application).

Also for shear-flow forces the forces may also not be fully homogeneous, for example since the flow speed near the side walls of a flow channel (*e.g*. with a rectangular cross section) may be lower than in the center of the flow cell (due to the no-slip boundary condition). By choosing a cross section with a high aspect ratio (low and wide) these flow effects may be minimized such that only a few percent of the cells experience a substantially smaller force than the cells in the center of the flow cell. Other methods to mitigate such effects and to specifically select cells that have experienced similar forces may include using flow cell geometries with multiple fluid inlets and/or outlets such that the properties of laminar flow can be used to ensure cells of interest only land in regions of homogeneous force and/or are only selected from regions of homogeneous force. In one example, by using three channel inlets side by side one can use the side channels as sheath flow channels to focus cells of interest inserted into the center channel where the acoustic and/or shear force may be substantially homogeneous. The sheath flow fluid may be the same buffer fluid as is used for the sample cells but then free of sample cells. By increasing the flow speed through the sheath flow channels the cells are more focused and confined to the center of the channel while by reducing the sheath flow speed the cells are allowed to spread out more. Similarly, on the collection side flows in three side-by-side collection channels may be controlled to possibly discard cells flowing close to the channel boundaries and only collecting cells from the center of the channel. By controlling the relative flow speeds of such side channels and the center channel asymmetrically the location of the effective interaction region of the sorting device can be further controlled and cells that have underwent defined forces can be selected and/or detected.

Further means to enable collection of cells from a specific interaction region (and therefore collection of cells that experienced a defined force) include means and methods wherein cells of interest may be provided with a photoactivatable label which may be subsequently activated by illumination with light of a suitable wavelength only in a well-defined interaction region of the device (*e.g*. near the center of a flow channel or in a center region under an (acoustic) force transducer) to photoactivate and/or switch the dye. Subsequently, the cells can be sorted and counted for example using fluorescence activated cell sorting (FACS) and only those cells which are activated are further used according to the methods described herein thereby obtaining the cells on which defined forces have been exerted. This may for example be highly useful for collecting cells that remained attached to the target cells. For example, the target cells and cells bound thereto may be trypsinized thereby obtaining both the target cells and cells that remained bound thereto in a suspension. Alternatively, attached cells can also be simply collected with physical means (*e.g*. scraping) from the area of interest, *i.e.* the surface area with a well-defined nominal force.

For centrifuge forces, it is easier to ensure that the force applied is homogeneous across the whole interaction region since such a force does not depend strongly on a location on a surface with respect to a force transducer and / or the wall of a flow channel or sample holder.

Accordingly, in connection to the subject matter disclosed herein, means and methods exist which allow one to exert forces on cells attached to a surface and collect the cells, detached and/or attached cells, on which defined forces have been exerted and determine *i.a*. the amount thereof, *i.e.* number of cells.

It is understood that with regard to the force exerted, the exact forces experienced by cells may also depend on cell size and or other cell properties such as density and compressibility. The force may be a nominal force and not the true force experienced by the cells carrying the receptor. *E.g*. it may be hard to precisely predict the average cell size, density, compressibility, etc. of the cells and the force may have been calculated based on theory alone or may have been calibrated using test particles with specific (preferably known) properties (see *e.g*. Kamsma, D., Creyghton, R., Sitters, G., Wuite, G. J. L., & Peterman, E. J. G. (2016). Tuning the Music: Acoustic Force Spectroscopy (AFS) 2.0. Methods, 105, 26-33). The force may be such a calculated or calibrated force expressed with units of N (*e.g*. pN) but it may also be expressed without calibration as the input power (Vpp) applied to a piezo element (see Sitters, G., Kamsma, D., Thalhammer, G., Ritsch-Marte, M., Peterman, E. J. G., & Wuite, G. J. L. (2014). Acoustic force spectroscopy. Nature Methods, 12(1), 47-50), as angular velocity squared (*ω*²) in the case of centrifugal force application or as flow speed v and or as shear stress (Pa) in applications using shear forces. As long as the forces exerted by the devices, *e.g*. shear force, acoustic force, or centrifugal forces, but not limited thereto, can be varied and controlled and reproduced in such devices such devices are suitable for the means and methods in accordance with the invention.

As the percentage of cells that remains bound at a certain applied force is indicative of cellular avidity, *i.e.* the larger the percentage of cells that is bound the higher the cellular avidity, it is useful to refer to such a percentage as a cellular avidity score. Of course, one may use a different measure which relates to cellular avidity. One may also refer to the percentage of detached cells instead, wherein conversely a low number is indicative of a relative higher cellular avidity. Instead of percentage, one may also provide the ratio of cells that remain attached divided by the total number of cells that interacted, or provide the ratio for detached cells. One may also, in case of a cellular avidity plot as shown herein determine the area under (or above) the curve. One may also, in case a fixed number of cells is to be provided to a target surface, simply provide the number of cells that have detached and/or remained attached. In any case, as long as a unit is provided that is representative of the number of cells that have detached or cells that have remained attached, relative to the total number of cells that have interacted, such a unit may be contemplated. Such a unit allows for ranking cellular avidities, when comparing e.g. different cells of interest. Providing such a unit may be referred to as providing a cellular avidity score.

Hence, instead of determining the percentage of cells that remain attached to the target cells, relative to the cells of the contacting step, in the means and methods any unit may be provided that is representative of the number of cells that have detached or cells that have remained attached, relative to the total number of cells that have interacted, as such a unit can be regarded as a cellular avidity score. A preferred unit of cellular avidity, to which also may be referred to as a cellular avidity score, may be the percentage of cells that remains attached, relative to the cells that have interacted, the latter being set at 100%. Thus, in a further embodiment, for each cell with a different expression level, the percentage of the total number of cells that remained bound relative to the cells that were contacted with the target cells attached to the surface is determined as a cellular avidity score. In yet another further embodiment, each of the cells with different expression levels are ranked based at least on the cellular avidity score. This way, when it is *e.g*. of interest to test the effect of cellular avidity with a defined receptor, constructs providing for different cellular avidities can be studied and compared.

With regard to providing cells having different expression levels, it is understood that this involves providing of each cell with a different expression level a plurality of cells such that a cellular avidity score can be determined. Hence, at least the steps of contacting and detaching of the method in accordance with the invention may be performed for each cell with a different expression level separately. For example, when a receptor of interest is identified and it is of interest to provide for cells with different cellular avidities therewith, different constructs may be generated with e.g. different promoter strengths or the like, and cells separately transduced therewith providing cells with different expression levels, and of each separately prepared transduced cells subsequently cellular avidity determined. It may be contemplated to combine cells with different expression levels. For example, each of the cells comprising different expression constructs for a receptor (and/or different copy numbers) may be provided with a different label which allows to distinguish the cells from each other. For example, utilizing different fluorescent labels, the steps of contacting and detaching may be performed with each of the cells with different expression levels combined. Detached and/or attached cells, may easily be analyzed with *e.g*. a FACS analysis. Hence, in one embodiment, each of the cells with different expression levels is provided separately and cellular avidity separately determined. In another embodiment, each of the cells with different expression levels are provided are differently labelled and subsequently combined and cellular avidity subsequently determined in steps c) - e) with the different cells combined. In another embodiment, each of the cells with different expression levels is provided with a different label, and at least the contacting step and force exertion step is performed with the different cells combined. In any case, as long as each of the cells with different expression levels may be identified, these may be combined.

It is understood that in accordance with the invention, when *e.g*. and preferably, cellular avidity measurements are performed with each of the cells with different expression levels of the receptors separately, and/or perhaps repeating cellular avidity measurements, this may involve utilizing the same target cells attached to a surface and/or may involve utilizing multiple provided target cells attached to a surface. For example, as shown in the examples, a chip (such as described i.a. in Fernández de Larrea, C., et al. (2020). Blood Cancer Discovery, 1(2), 146-154, WO2018083193, and such as available from LUMICKS for the z-Movi^{®} device (see *i.a*. z-Movi^{®}-Brochure_2021.pdf, available from <https://lumicks.com/products/z-Movi-cell-interaction-studies/#brochure>) with target cells attached to a surface may be repeatedly used in the z-Movi^{®} device and cells that have remained bound to the target cells and remain bound to the target layer in a subsequent measurement may *e.g.* be masked in analysis (*e.g.* by the software or by manually identifying these cells and excluding these in a subsequent measurement). Hence, it is understood that repeating the methods may be performed with the same target cells attached to a surface or may be performed with further provided target cells attached to a surface. When multiple surfaces with attached target cells are provided, it is understood that of course these are prepared in the same way such that different measurements with each of the multiple surfaces can be compared and are substantially reproducible. Hence, in one embodiment, the method for determining cellular avidity is repeated with the same target cells attached to a surface, *e.g*. as a monolayer.

It is understood that a synapse is a specialized structure that forms when the plasma membranes of two cells come into close proximity to transmit signals. Synapses can form between cells expressing a receptor and target cells, when *e.g*. the cells with the receptor are effector cells. Cells of the immune system form synapses that are essential for cell activation and function. Lymphocytes such as T cells, B cells and natural killer (NK) cells form synapses that can be referred to as immunological synapses. Such a synapse typically forms between effector cells and target cells, *e.g*. cells presenting an antigen. A non-limiting example is *e.g*. a T cell or a CAR-T cell and a cancer cell. The formation of synapses between *e.g*. an effector cell and a target cell, for example an APC (antigen presenting cell), is a hall-mark event and signals the presence of specific interactions (*i.e.* the specific interaction between, for example, a TCR or CAR and an antigen recognized thereby) between the effector cell and the target cell that are involved in the formation of such immunological synapses.

In the case of a T cell, a synapse can be formed between the lymphocyte and antigen-presenting cells (APCs) during the recognition of the peptide antigen-major histocompatibility complex (pMHC) ligand by the T cell antigen receptor (TCR). The TCR and pMHC are both membrane-bound so the TCR will only be triggered by its ligand at the interface between T cells and APCs. A synapse can be observed at the T cell - APC interface as concentric rings by confocal microscopy, often referred to as "bull's eye" (Huppa, J. B., & Davis, M. M. (2003). T cell-antigen recognition and the immunological synapse. Nature Reviews Immunology, 3(12), 973-983). These rings were named the central, peripheral, and distal supramolecular activation cluster (i.e.respectively cSMAC, pSMAC and dSMAC). The TCR has been reported to be present in the cSMAC, whereas other lymphocyte specific proteins such as lymphocyte function-associated antigen-1 (LFA-1), are integrated into the pSMAC ring that surrounds the TCR. The formation of this ringed structure ("bull's eye") is however not universal and other formations such as "multifocal immunological synapses" between T cells and dendritic cells, or the like, have been described.

The immunological synapse can be considered to be any structure formed at the interface resulting from a functional and specific effector-target cell interaction, such as for example T cell-APC contacts. Markers associated with effector cells and synapse formation include one or more of CD43, CD44, CD45, LFA-1, Talin, F-actin, ZAP70, CD2, CD4, CD8, CD3, CD28, PD-1, ICOS, and TCR. Markers of target cells include one or more of ICAM-1 (associates with LFA-1), CD48/58 (interacting with CD2) CD80/CD86 (interacting with CTLA-4 and CD28), PDL1/PDL2 (associating with PD-1), and MHC presenting the antigen (that specifically interacts with the TCR). These markers, and concentrations thereof, may be detected *e.g*. with fluorescent labels at the interface between effector cell and target cell, or intracellularly in close proximity to the synaptic interface.

For example, markers of effector cells that have been associated with dSMAC are CD43, CD44, CD45. The effector cell markers LFA-1, Talin, F-actin, CD2, CD4 and CD8 have been associated with pSMAC. The markers CD3, CD28, PD-1, ICOS, and TCR of effector cells have been associated with cSMAC. Markers that have been associated with a synapse on a target cell are ICAM-1 (associates with LFA-1), CD48/58 (interacting with CD2) CD80/CD86 (interacting with CTLA-4 and CD28), and PDL1/PDL2 (associating with PD-1), and of course an MHC presenting the antigen (that specifically interacts with the TCR). These markers have been shown to be associated with synapses in a TCR-MHC interaction. Likewise, cell engagers (such as a bispecific antibody that *e.g*. binds CD3 on an effector cell with one arm and with the other arm an antigen on a target cell) which engage an effector cell with a target cell, can trigger the formation of a similar synapse structure as observed with a classic TCR-MHC interaction.

With regard to CARs of *e.g*. CAR T cells, these are chimeric antigen receptors that are to mimic a TCR or the like. CARs are engineered. The first generation of CAR were provided with an antigen recognition part often an antibody derived region (e.g. a scFv) fused to a transmembrane region and intracellular region of *e.g*. a CD3 ζ-chain. Later generations combined intracellular signalling domains from various costimulatory protein receptors (*e.g*., CD28, 41BB, ICOS) incorporated in the cytoplasmic tail of the CAR to enhance signalling further. Further generations also incorporated in their design an inducible release of transgenic immune modifiers, such as IL-12, to shape the tumor environment by augmenting *e.g*. T cell activation, attracting and activating innate immunity. As CARs often have antibody variable regions incorporated, these can target *e.g*. receptors themselves that are presented at the surface of a cell (*e.g*. Her2, PD-1 etc.), or can also target antigens presented by MHC, derived *e.g*. from proteins intracellular processed by the ubiquitin-proteasome system. Such peptides presented by MHC include proteins that are processed internally and presented by MHC, which can be derived from receptors, secreted proteins, intracellular proteins or internalized proteins. Synapses formed between CARs and target cells may not provide a classical bull's-eye like structure with a well-characterized SMAC domain, but may result in less organized pattern. Multiple CAR microclusters form and signalling molecules, which are dispersed in the center of the synapse interface.

Synapse formation is a spatiotemporal process that starts *e.g*. by TCR binding to MHC or binding of an antigen with CAR or cell engagement, and subsequent phosphorylation of the cytosolic tails of CD3 resulting in a triggered state. This sets of a cascade of processes that result in an activated T cell state, which also depends on the effector cell type and its phenotypic state. Key processes include: calcium signalling, internal cell structure and/or cytoskeleton changes of effector cells, involving F-Actin, Talin, and changes in microtubules, centrosomes, lytic granules, nucleus position, and mitochondrial location. Transactivation of adhesion molecules, cytokine and marker expression. IFNγ, granzyme and perforin may be released by effector cells to thereby induce *i.a*. target cell killing. Also, in addition, in target cells apoptotic markers can be found, including ICAM-1 clustering, phosphatidyl translocation, mitochondrial depolarization, caspase-3 activation and DNA fragmentation. Hence, various stages of specific effector cell and target cell interaction and immune activation can be detected.

In any case, synapse formation, or a synapse can be determined by staining, *i.e.* with fluorescent labels, ligands, antibodies, or probes, or the like, which may be used on live cells or on fixed cells targeting the mechanisms involved in T cell activation and/or synapse formation. Sequencing based methods may also be used to identify activated T cells and thereby associate mRNA levels with the formation of stable synapses. T cell activation leads to changes in mRNA stability and expression. *E.g*. increases in expression of cytokine or secretory transcripts (IL2, IFNγ, granzyme, perforin) and proliferation pathways and either bulk or single-cell RNA sequencing may be used to detect these changes and correlate these to the number or synapse formed.

In any case, in the cells obtained, which can be either in the form of target cells bound to effector cells or separated cells, *e.g*. with trypsin, the marker associated with synapse formation can be determined. Thus, in a further embodiment, the marker associated with synapse formation is determined in either the target cell or the effector cell. In another embodiment, in the methods in accordance with the invention one or more markers associated with synapse formation are determined and the one or more markers are determined in the effector cells and/or in the target cells. When cells are labelled, either in a singlet state or doublet state, cells may be highly advantageously be sorted and collected and subsequently analysed.

In one embodiment, a method in accordance with the invention is provided, wherein the marker associated with synapse formation is selected from the group consisting of calcium signalling signatures; spatial clustering of synapse localized molecules such as LFA-1, CD28, CD3, Agrin; changes to internal cell structure and/or cytoskeleton such as F-Actin, Talin, microtubules, centrosome, lytic granules, nucleus position, mitochondrial relocation; changes in effector cell motility; changes in external cell morphology and/or cell shape; and apoptosis of target cells. Synapse formation includes the initiation of a synapse up to and including the establishment of a synapse in which, as described above but not necessarily limited thereto, markers associated with synapse formation are associated.

In a further embodiment, the marker for synapse formation is calcium signalling, which can be detected with fluorescent calcium indicators, such as Fura2 AM (available from Invitrogen, item nr. F1221), which marker is suitable for detection in effector cells and in live cells. Other suitable dyes to detect calcium signalling can be selected from the group of Fura Red AM, Indo-1, pentapotassium, Fluo-3, fluo-4, Calcium Green-1, Rhod-2 and X-Rhod-1, Oregon Green 488 BAPTA. Hence, in one embodiment, the marker for synapse formation is calcium signalling which is detected with an indicator selected from the group consisting of Fura2 AM, Fura Red AM, Indo-1, pentapotassium, Fluo-3, fluo-4, Calcium Green-1, Rhod-2 and X-Rhod-1, and Oregon Green 488 BAPTA. With these markers calcium signalling can be detected which is a hallmark of synapse formation.

Furthermore, membrane potential dyes may also be used a calcium signalling indicators, such as the Invitrogen FluoVolt^{™} Membrane Potential Kit (Catalog number: F10488). Depolarization of the synapse forming cells by using a slow-response potential-sensitive probe such as Invitrogen DiSBAC2(3) (Bis-(1,3-Diethylthiobarbituric Acid)Trimethine Oxonol), Catalog number: B413. Hence, in another embodiment the marker for synapse formation is detected utilizing membrane potential dyes.

In another embodiment, the marker for synapse formation is cytoskeleton rearrangement, which can be detected in effector cells with live or fixed cells, using cell staining, e.g. F-actin can be detected with Phalloidin conjugates or CellMask^{™} (Invitrogen, item nr A57243). Other usable stains can include SiR-Actin, CellLight^{™} Talin-GFP, BacMam 2.0, or Tubulin Tracker Deep Red. Hence, in one embodiment, the marker for synapse formation is cytoskeleton rearrangement, which is detected with a stain selected from the group consisting of Phalloidin conjugates, CellMask^{™}, SiR-Actin, Cell Light^{™} Talin-GFP, BacMam 2.0, or Tubulin Tracker Deep Red. With these markers, cytoskeleton rearrangement can be detected.

In another embodiment, the marker for synapse formation involves monitoring effector cell motility. Detection of effector cell motility can be performed by video/timelapse monitoring of effector cells that remain in contact with the target cells after the force has been exerted. Detection of synapse formation includes detecting effector cell immobility, *i.e.* upon synapse formation effector cells will stop moving and remain into contact with the target cell with which it forms a synapse. Cell motility can be detected by membrane staining of effector cells and imaging, or using brightfield, darkfield or phase contrast microscopy.

It is understood that for some of the methods for detecting a marker for synapse formation which use *e.g*. microscopy and monitoring of motility, or short-lived signals, may be combined with having cells provided with e.g. photoactivatable label, to, upon detection of the marker, activate such a label in the cell, such that in subsequent steps, e.g. sorting, FACS analysis or the like, cell for which the marker associated with synapse formation was detected can easily be tracked.

As described above, in accordance with the invention, markers associated with synapse formation can be determined, e.g. via utilizing labels or the like. However, it may be highly advantageous to sequence obtained cells and identify synapse formation by sequencing. It is understood that sequencing comprises nucleotide sequencing, *e.g*. sequencing of DNA and/or RNA as expressed in cells. Means and methods are widely known in the art to sequence DNA and/or RNA as expressed in the cell. Hence in another embodiment, in the methods in accordance with the invention the markers are determined with sequencing. This is in particular highly useful for such markers which are up- or downregulated in target cells and/or effector cells in forming a synapse or having a synapse. Suitable markers for T cell activation and synapse formation are transcripts linked to interferon expression, proliferation, and cytokine expression and include: Interferon pathway upregulation: CD4, IFIT3, IFIT2, STAT1, MX1, IRF7, ISG15, IFITM3, OAS2, JAK2, SOCS1, TRIM21; proliferation: LIF, IL2, CENPV, NME1, FABP5, ORC6, G0S2, GCK; cytokine expression: CCL3, IFNG, CCL4, XCL1, XCL2, CSF2, IL10, HOPX, TIM3, LAG3, PRF1, TNFRSF9, NKG7, IL26. Sequencing may also be used to identify non-synapse forming cells by detecting molecular signatures linked to resting cell states: FOXP3, CTLA4, MTNFRSF4, IRF4, BATF, TNFRSF18, TOX2, PRDMI, LEF1, ATM, SELL, KLF2, ITGA6, IL7R, CD52, S100A4, TGFB3, AQP3, NLRP3, KLF2, ITGB7.

It may be also advantageous to determine the molecular state of the target cells as this can give an indication of the cell killing potency of the effector cells. In the case of paired analysis were effector-targeT cell doublets are recovered this enables direct linking of effector cell phenotype with their killing capabilities. Next to staining methods for apoptosis commonly used in the field sequencing or qPCR can be used to detect transcripts linked to apoptosis or cell survival in the target cell, markers include: Bcl-2 family (BCL-xL) caspases 3, caspases 7, cleaved PARP, bax, bad, bak, bid, puma noxa, bcl-2, bcl-xl, mcl-1, p53, and cytochrome c, Smac/ Diablo, survivn, Mcl-1, RNA Y1.

As it is understood that changes in gene expression may take some time and are not necessarily immediately detectable, one may allow after the step of exerting the force, the cells to remain bound to the target cells for some time and have these remain attached to the surface as well. Alternatively, one may also separate e.g. doublets that remained after exerting the force and subsequently e.g. sort doublets in single wells, and allow these to remain for some time. A suitable time to allow for expression of markers associated with synapse formation may be from 1 hour to 24 hours.

This is highly advantageous as sequencing methods allow for single cell sequencing, which also allows for determining e.g. the sequences of receptors expressed by the cell, or designed expression constructs, as well. Hence, in a further embodiment, different receptors or different expression constructs are determined and identified via sequencing. In yet another embodiment, sequencing comprises single cell sequencing. In still another embodiment, sequencing comprises sequencing the expressed genome. Sequencing the expressed genome is highly useful as it allows to determine up- and downregulated gene expression. Nevertheless, sequencing genomic DNA may be useful as it may also provide useful information *e.g*. epigenetic markers associated with *in vivo* potency and durable responses.

With regard to the methods for differentiating between cells having different expression levels of receptors, e.g. due to construct design or copy number or due to different subpopulations of effector cells, while expressing the same sequence, in view of the above, in the scenario wherein these cells are effector cells capable of forming a synapse, a further differentiation can be made when taking into account markers associated with synapse formation. Hence, in a further embodiment, the cells having different expression levels of a receptor are effector cells, and wherein in cells with different expression levels and bound with target cells after the step of applying the force the presence of a marker associated with synapse formation is determined. It is understood that when such markers are determined, and cellular avidity scores determined, cellular avidity scores can be determined based on the number of effector cells with different expression levels and with the marker associated with synapse formation. A cellular avidity score, which takes into account target cells and effector cells bound to each other and having a marker associated with synapse formation may provide for a more accurate cellular avidity score which is more reflective of the function of *e.g.* effector cells, *i.e.* forming synapses with target cells. Hence, such a cellular avidity score may also be referred to as a functional cellular avidity score or a synaptic cellular avidity score, and it is understood that where herein cellular avidity scores are determined in accordance with the invention, this may also include cellular avidity scores taking into account synapse markers. This way, effector cells expressing different expression levels of a receptor may be defined further, *e.g*. effector cells with a defined expression level that remained bound, may be further defined as comprising effector cells with a defined expression level that remained bound having a synapse, and effector cells with a defined expression level that remained bound not having a synapse. Accordingly cellular avidity scores can be determined by calculating e.g. the ratio of the number of cells of an effector cell with a defined expression level that were bound to target cells after applying a force and associated with a synapse to the number of said effector cells with a defined expression level initially provided, or to the number of effector cells with a defined expression level that remained bound plus the said effector cells with a defined expression level that moved away from the target cells.

In addition to providing control over cellular avidity by modulating receptor expression, as shown in the example section, cells having the same receptor but varying in cellular avidity in cell population may be advantageously enriched based on their expression level by using force separation methods. This way, the fraction of a subpopulation comprised in the cell population, having *e.g*. low or high expression, may be enriched for (or, conversely reduced). Hence, in one embodiment, a method is provided for enriching a particular cell population, comprised in a population of cells comprising cells with different expression levels of a receptor, said particular cell population having a relatively higher or lower expression level of the receptor; comprising the steps of:
- providing target cells attached to a surface ;
- contacting a population of cells with different expression levels of a receptor with the target cells attached to the surface to allow the population of cells to interact with the target cells;
- exerting a force on the population of cells with different expression levels of the receptor;
- collecting cells from the cell population which detached from and/or remained attached to the target cells attached to the surface, therewith providing fractions comprising enriched cell populations comprising a relatively higher or lower expression level of the receptor.

As expression level may correlate with copy number, *e.g*. the number of vector genome copies integrated per cell, such methods also allow to enrich from a cell population with different copy numbers, a subpopulation of copy number(s). Thus, in a further embodiment, a method is provided for enriching a cell population comprising a range of copy numbers of a gene construct expressing a receptor, said method comprising:
- providing target cells attached to a surface ;
- providing a cell population comprising different copy numbers of the gene construct expressing the receptor; said copy numbers ranging from 1 to 2, or more;
- contacting the cell population with the target cells attached to a surface;
- exerting a force on the cell population;
- collecting cells from the cell population which detached from and/or remained attached to the target cells attached to the surface, therewith providing fractions comprising enriched cell populations comprising a relatively higher or lower copy number of the gene construct.

Hence, as shown in the example section, by exerting a force, cells having different expression levels of receptors due to *e.g*. copy numbers, can provide for different cellular avidities and based on this difference, cells may detach from and/or remain attached to target cells at different rates at defined forces. By *e.g.* repeating the enrichment process, highly enriched cell fractions can be advantageously obtained. This may be for example be highly advantageous in a scenario wherein a cell fraction is to be prepared for administration to a subject, *e.g*. a patient. This way, *e.g.* cells having *e.g.* predominantly a single copy can be obtained, which may be advantageous when e.g. higher copy numbers are associated with reduced efficacy, exhaustion or side effects.

Enriched cell population thus obtained accordingly with means and methods in accordance with the invention may subsequently in a final step be admixed with a pharmaceutically acceptable buffer or otherwise pharmaceutical acceptably formulated.

In one embodiment, the enriched fraction thus obtained is enriched with cells having a copy number of 1. Of course, suitable means and methods may be selected to provide for optimal enrichment, *e.g*. of other fractions being enriched for another copy number *e.g*. 2 and/or related to different expression levels or ranges of expression levels.

Any suitable force application method may be contemplated in accordance with the invention. Increasing the force can be well controlled with acceleration based methods of applying force such as centrifugation, with shear flow and with acoustic force, which are all suitable means to be used in the methods in accordance with the invention but any other means of controllably causing a force on the cells attached to the target cells thereby forcing them away from the target cells or the functionalized wall surface, may be contemplated. In any case in any of the methods as described and as outlined herein, and as described above, the applied force may be an acoustic force, a shear flow force or an acceleration force such as a centrifugal force. In a further embodiment, embodiment, the applied force in the means and methods of the invention is a force ramp, preferably a linear force ramp. It is understood that the different forces that are to be applied can be constant forces applied for a defined period. The forces applied may be in various forms as a function of time. Preferably however, the applied force is an increasing force, that is, after the incubation step, an increasing force is applied for a defined period until a defined end force is reached. For example, as shown in the example section, with e.g. an acoustic force in about 150 seconds, a defined end force is reached of 1000 pN. Increasing the force is preferably done in a linear force ramp, but other ways to increase the force over time may also be used (*e.g*. exponential loading where the force is doubled over a certain time period and keeps doubling until a defined end force is reached). Hence, in a preferred embodiment the applied force is a force ramp, preferably a linear force ramp.

As said, any suitable material may be selected for attaching the target cells. Preferably, the surface may be glass or plastic, as such materials are known to be highly suitable for attaching cells and may allow for visual inspection of the attached cells and cells carrying a receptor interacting therewith. The target cells that are attached to the surface, preferably are attached as a monolayer. The monolayer preferably is at high confluency. The subsequent cells that are to interact with the target cells are preferably provided in a relatively low cell density as compared with the target cells, such that substantially all cells that are to interact with target cells are outnumbered (there are more target cells). Such provides for advantageous controllable conditions when applying the force on the cells that interact with the target cells.

In one embodiment, the target cells present a cancer antigen, and/or the target cells are cancer cells. It is understood that cells presenting an antigen may include *e.g*. an MHC complex presenting an antigen. Target cells, and in particular cancer cells, may also (over) express other differentiating molecules on their surface. Hence, in any case, as cancer cells, or cells presenting a cancer antigen, are of particular interest, such cells may be highly preferred. Nevertheless, other target cells for which it is of interest to provide cells with a receptor that can bind to target cells, (*e.g*. as outlined in Figure 10) modulate receptor expression and/or select particular cells carrying a receptor with a defined range of expression and/or copy number can be of interest as well.

With regard to cells carrying a receptor, of particular interest in the art and in accordance with the invention are effector cells, *i.e.* effector cell of the immune system that can exert specific actions towards certain target cells. Suitable target cells which can be selected from the group consisting of T lymphocytes, NK cells, monocytes, macrophages and dendritic cells. From such cells, clonal populations may be identified that are of interest, e.g. expressing a particular receptor, and of which variants can be made by genetically modifying these such that expression levels may be varied and different cells with different expression levels may be obtained. Highly advantageously, such cells may also be transduced with vectors designed to express receptors, e.g. expressed from differently designed constructs and/or such cells may be transduced with different copy numbers of such designed vectors. Receptors that may be contemplated and preferably expressed include *e.g*. a CAR receptor, a TCR, and my also include a stimulatory or inhibitory coreceptors, or a receptor that can be engaged via a bispecific antibody.

In another embodiment, as the cell carrying a receptor on its cell surface is to interact with the target cell, *e.g*. via a ligand presented on its cell surface, it is also understood that there may be agents that are capable of modulating this interaction and of which it is of interest to include these in the steps of contacting and force exertion steps in the methods as described herein.

It was observed that when cells that remained bound and attached after performing a cellular avidity method were resuspended utilizing *i.a*. repeated pipetting and thus exerting a significant force, cells attached to the surface were detached and moreover, a portion of the cells that were bound to the attached cells became unbound. Hence, this implied that with such a process step a differential force can be applied on bound cells that can exceed the maximum force that is applied away from cells attached to a surface. This way, further cell-cell bonds that may be aspecific cell-cell bonds can thus be broken therewith providing substantially specific cell-cell bonds that remain, *e.g*. effector cells bound with target cells that formed a synapse can be retained. Hence, in a further embodiment, after the force has been applied away from the attached cells, subsequently, the cells are resuspended and a differential force is applied such that substantially effector cells that are bound to each other via a synapse remain bound to each other and substantially cells that have formed aspecific bonds are unbound, *i.e.* have their cell-cell bonds broken. It is also understood that instead of applying the force away, the differential force may be applied, and that by applying a differential force, it may no longer be required to have cells attached to a surface, *i.e.* immobilized. Furthermore, it is also understood that after the differential force has been applied, cells are highly preferably separated and/or sorted in order to avoid further interaction between cells to avoid establishing additional cell-cell bonds. Hence, highly preferably, cells are collected and sorted and/or analysed after the differential force has been applied. As described herein above, like in the scenario of applying a force away from target cells, by applying such a differential force, one can differentiate between cells having different levels of expression of a receptor. Cellular avidity scores can be determined, optionally combined with determining the presence of markers associated with synapses. This way, cellular avidity scores can be assigned to cells with different expression levels. This way, cell populations having different expression levels can be enriched for, *e.g*. as having defined copy numbers and/or defined expression levels, as well.

As is clear from the above, the type of force that is to be applied in accordance with the invention is a force capable of breaking cell-cell bonds, *i.e.* the force exerted causes cells bound to each other move away from each other to such an extent that a cell-cell bond may break or rupture. A differential force means that the force on one cell differs from the force on the other cell with regard to direction of the force and/or the magnitude of the force, resulting in a net force allowing to break cell-cell bonds if the differential force exceeds the binding force.

For example, when a target cell bound to an effector cell, a doublet, is forced through a nozzle, the closer to the throat of the nozzle the faster the flow. This means that the first cell to enter the nozzle is subjected to a stronger acceleration than the cell lagging behind and the cells experience a differential force resulting in a net force which can result in cell-cell bond rupture, provided the force is large enough (see *e.g*. Figure 11, in particular 11d). A differential force that can be applied includes a shear force, e.g. such as can be applied utilizing repeated pipetting (repeated upwards and downwards flow of the sample) or flow through a nozzle.

Other means and methods are known in the art with which shear forces can be applied to cells, e.g. flowing a cell suspension at a constant speed and bombarding these cells to a flat surface at a defined angle. Furthermore, forcing a cell suspension through a needle with a defined internal diameter and a defined force may provide for a well controllable shear force as well. The cell suspension may be subjected to several rounds of such process steps to ensure substantially all cell-cell bonds experience the maximum force that may be achieved with the process step. Such a process step allows for automation, enabling control and repeatability of the process, therewith controlling shear forces exerted. Suitable devices for breaking apart cell-cell bonds which are not synapses are known in the art (*e.g*. Zahniser et al., J. Histochem. Cytochem. 1979, 27 (1), 635-641). Also, by properly tuning the forces in a flow-cytometer normally used to measure cell deformations, such as *e.g*. described in Otto, et al. Nat. Methods 12, 199-202 (2015) suitable forces can be applied. Tuning can be achieved *e.g*. by changing the nozzle size or geometry and/or the flow speeds used. Other suitable devices known in the art may include for a vortex mixer, with which shear forces may be suitably applied as well. Accordingly, in one embodiment, the force applied involves a shear force.

In another embodiment, the force applied is an ultrasonic force. It is understood, as outline above, that such ultrasonic forces are not forces such as applied *e.g*. in a device as available from Lumicks, wherein the force is away from attached cells (*e.g.* such as in the LUMICKS z-Movi^{®} Cell Avidity Analyzer, *e.g.* as used by Larson et al., Nature 604, 7906:1-8, April 13, 2022). It is also understood that the ultrasonic force is selected such that cells are not lysed. Hence, appropriate ultrasonic forces can be applied to cells such that cell-cell bonds can be ruptured, which more preferably includes breaking aspecific cell-cell bonds and less preferably breaks specific cell-cell bonds in which an immune synapse is formed. Examples of using ultrasonic forces to break (aspecific) cell-cell bonds, are known in the art (*e.g.* as described in Buddy et al., Biomaterials Science: An Introduction to Materials in Medicine, 3rd edition, 2013, Chapter II. 2.8, page 576; and Moore et al., Experimental Cell Research, Volume 65, Issue 1, 1971, Pages 228-232).

In any case, suitable applied forces which are known in the art include *e.g.* a force in the range of 50 pN - 10 nN, which said force is a net force exerted on one cell relative to the other cell, of two cells bound to each other. Which means the force is exerted on the cell-cell bond. In another embodiment, the force exerted on one of the two cells relative to the other cell is at least 50 pN, or at least 100 pN, or at least least 200 pN. In another embodiment, the force exerted is at most 10 nN, at most 5 nN, at most 3 nM, at most 2 nM, or at most 1 nN. In yet another embodiment, the force is selected from the range of 1 pN - 10 nN, from 100 pN - 10 nN, from 500 pN - 10 nN, from 1 nN - 10 nN. In still a further embodiment, the force is selected from the range of 500 pN - 5 nN, from 500 pN - 4 pN, from 500 pN - 3 pN. For example, a suitable amount of force that can be exerted between cells (*e.g.* such as in the z-Movi^{®} device) can be selected to be in the range of 200 pN - 3000 pN. Of course, these force ranges are known to be useful with cells attached to a surface, and the maximum force that may be selected may exceed 3000 pN as it may not be required to have the cells attached to a surface.

Accordingly, it is understood that in these embodiments, the differential force to be applied does not require either of the target cells or the cells having different expression levels of a receptor, *e.g.* comprising a range of copy numbers of a gene construct expressing a receptor, to be attached, and the differential force can be a force selected from the range of 50 pN - 10 nN. In another embodiment, in methods in accordance with the invention wherein the force that is applied is a differential force, neither the target cells nor the cells having different expression levels of a receptor, require to be attached to a surface, and the differential force is applied in the range of 50 pN - 10 nN, thereby providing cells substantially comprised of target cells, cells having different expression levels of a receptor, and cells having different expression levels of a receptor bound to target cells. In another embodiment, in methods in accordance with the invention wherein the force that is applied is a differential force, neither the target cells nor the cells having different expression levels of a receptor, require to be attached to a surface, and the differential force is applied in the range of 50 pN - 10 nN such that formed cell-cell bonds of cells with a receptor with a low copy number are broken, *e.g.* wherein the low copy number is 1. It is understood that cells with a receptor bound with target cells can have different strengths of binding, *e.g.* due to a certain copy number or certain expression level, and by selecting an appropriate differential force and performing the methods in accordance with the invention, one can enrich for cells with receptors having certain copy numbers or expression levels.

Without being bound by theory, it is understood the force required to break an aspecific cell-cell bond versus a specific cell-cell bond that forms *e.g.* a synapse, differs. Likewise, with different expression levels of receptors, the cell-cell bonds that can be formed can differ as well with regard to binding strength, *i.e.* due to receptor density on the cell surface. Hence, when performing *e.g.* the methods of the invention with effector cells, at the one end of the spectrum a strong synapse can be formed and at the other end of the spectrum, weak binding occurs without forming a synapse, all occurring with *e.g.* the same receptor sequence. The difference in binding strength can be to such an extent that the ranges of the required forces to break a cell-cell bond resulting from different levels of expression do not overlap. It is understood that some overlap may occur. In case there is substantially no overlap, a differential force can be selected which allows substantially for certain cell-cell bonds to break, *e.g.* because of having a low level of expression, while on the other hand substantially retaining cell-cell bonds that formed a strong cell-cell bond, such as a synapse, *e.g.* due to higher expression levels. In any case, with or without overlap, by applying a differential force one can enrich cells expressing a receptor with regard to expression levels, and the amount of enrichment depends on the binding strength and level of (differential) force applied.

Accordingly, in one embodiment, in the methods in according with the invention, wherein after the step of applying the force, the cells are resuspended and a differential force is applied such that formed aspecific cell-cell bonds are broken. In another embodiment, in the methods in according with the invention, after the step of applying the force, the cells are resuspended and a differential force is applied such that formed cell-cell bonds of cells with a receptor with a low copy number are broken, e.g. wherein the low copy number is 1. In another embodiment, in the methods in according with the invention, instead of applying the force away from the target cells, a differential force is applied such that formed cell-cell bonds of cells with a receptor with a low copy number are broken, *e.g.* wherein the low copy number is 1. In another embodiment, in the methods in accordance with the invention, in the step of applying a force on the cells with different expression levels, instead a differential force is applied. In a further embodiment, neither the target cells nor the cells with different expression levels are required to be attached to surface in the steps of the method. In another further embodiment, in the methods in accordance with the invention the cells having different expression levels of a receptor are effector cells and the differential force is such that cells that are bound via a synapse remain bound to each other and formed aspecific cell-cell bonds are broken.

### Examples

### Example 1

### Materials and methods

### Effector cell preparation

Untransduced or FMC63-transduced primary CAR T cells were obtained from ProMab Biotechnologies Inc. (Anti-CD19-TF-CD28-CD3z (PMC-152)). Two days before the avidity experiments, primary T cells (untransduced (UNT) and CAR transduced) were thawed and purified. UNT and CAR T cells were derived from the same donor. After thawing, a fraction of the CAR transduced T cells was purified using magnetic activated cell sorting (MACSBeads, Miltenyi) using an anti-flag antibody. The concentration of antibody was titrated to enrich for the T cells with the highest CAR expression. This was the CAR High T cell population. The flow-through was also collected. This was the CAR Low T cell population. The four populations: UNT, CAR unpurified, CAR High and CAR Low T cells were put in culture with interleukin 2 (IL2) supplement for two days in preparation for the avidity experiment. These were used as the effector cells.

### FACS analysis

The effective transduction of the cells and the MACS purification procedure to obtain samples with low and high expression levels of the CAR receptor were checked using standard FACS analysis. The relevant FACS plots are shown in figure 1. When looking in the FLAG-PE PE-A channel it is clear that the transduced CAR -T population (CAR unpurified) contained (almost) no untransduced cells but did contain at least two distinct populations with lower and higher expression levels of the CAR (seen as two peaks in the graph). The MACS separation resulted in two distinct fractions: one fraction with (almost) purely the low expression level (CAR low) and another fraction with predominantly the higher expression level (CAR high).

### Target cell monolayer preparation

z-Movi^{®} chips, channel slides (Ibidi, #80166) and 18mm circular glass slides were treated with NaOH 1 M for 1 hour, rinsed with water and let dry in a dry 37°C incubator. Subsequently, after 1 hour, the chips and slides were coated with Poly-L-Lysine (#P4707-50 ML, Sigma) diluted 1:5 in PBS for 10 minutes at room temperature and left to dry overnight in a dry 37°C incubator. The following day Nalm6 (CD19+) or HeLaCD19 + cells were used for seeding monolayers of target cells. For the 18mm circular glass slides, a 4-well silicone insert (Ibidi, #80489) was stuck to the PLL-treated glass surface prior to seeding. For both Nalm6 and HeLa cell lines, cells were counted and seeded in serum-free medium on the different vessels at a concentration required to achieve a monolayer confluence close to 100%. For the Ibidi channel slides the monolayers were seeded on the ceiling of the channel (see Figure 5). 60 µl of the target cell suspension was pipetted into the inlet and seeded by gravity by tilting the slide for few seconds until the fluid reached the outlet. 60 µL is the volume of the channel, so this way the whole channel is filled, and air plugs in inlet and outlet keep the liquid from moving. The slide was incubated upside down in a humidity and CO₂ controlled incubator for 30 minutes. Medium was exchanged by applying 60 µl of new medium to the inlet and withdrawing 60 µl from the outlet, 4 times. The slide was put back upside down in a humidity and CO₂ controlled incubator.

The vessels were placed for 30 minutes either in a dry incubator (z-Movi^{®} chips) or in a humidity and CO₂ controlled incubator (channel slides and circular glass slides), then the medium was exchanged with a serum-containing one. The chips were incubated for an additional hour before the avidity experiment. For the HeLa cells seeded on 18mm circular glass slides,. cells were kept overnight in a humidity and CO₂ controlled incubator.

### z-Movi^{®} acoustic force avidity measurements

The effector cells were stained with CellTrace^{™} Far Red (Thermo Fisher Scientific, cat. # C34564) at 1 µM for 15 minutes in PBS at 37°C and then resuspended at 10 million/mL in complete medium and used for the avidity experiments. The stained effector cells were introduced in the target cell-seeded flow cell, incubated for 5 minutes, and then a 1 to 1000 pN force ramp was applied throughout 2.5 minutes using a z-Movi^{®} device operated with the Oceon V1.2 software. All cell types were run multiple times (2 runs/sample/chip using two different chips). Samples were run in random order sequentially on the same monolayer. The avidity experiment analysis was performed using Oceon V1.2.

### Centrifuge experiments

The effector cells were stained with different CellTrace^{™} dyes (Thermo Fisher Scientific) at 1 µM for 15 minutes in PBS at 37°C. The following stains were used to label the cells with different colors: CAR High: Far Red (cat. # C34564); UNT: Violet (cat. # C34557); CAR unpurified: Yellow (cat. # C34567); CAR Low: CFSE (cat. # C34554). The different cells were mixed in a cell suspension containing equal amounts of the samples for the experiments with the Ibidi channel slides or run separately with one sample per well on the 18 mm glass slides. Images were acquired with a Nikon Ti2 fluorescence microscope. Data was analysed using FlowJo Particles Counter after image processing (for 18mm glass slides experiments) or NIS Elements software (Nikon) (for channel slides experiments).

For the experiment with the circular 18 mm slide with the 4 well insert the slide was placed on a 3D printed slide holder that fits into a 50mL tube (see Figure 2). The slide was placed with the monolayer facing towards the bottom of the tube. The slide and holder were placed inside a 50 mL tube filled with cell medium. The tube was closed off with a cap, placed into a centrifuge, and spun for 2 minutes at 1000xg. This removed the fraction of the target cells that were not well attached to the glass. The slide was then taken out of the tube and the effector cells were pipetted on top of the monolayer and incubated for 5 minutes in a humidity and CO2 controlled incubator. Each effector cell sample was incubated on a separate well. One image of each well was taken using the fluorescent microscope. Each image had Brightfield, FarRed, Violet, Green channel or Yellow channels (depending on whether CAR unpurified or CAR low were used). The slide was put back into the tube + holder, and spun with the centrifuge at 250xg for 2 minutes. The slide was taken out again and one image of each well was taken using the fluorescent microscope. These steps were repeated at increasing speeds to 500xg, 1000xg, 2000xg. Here g indicates the g Force, also known as the relative centrifuge force (RCF).

For experiments using the Ibidi channel slides the slide was placed into an adaptor (see image 4) for the centrifuge bucket and spun for 2 minutes at 1000g. This removed the target cells that were not well attached to the glass. The slide was taken out of the centrifuge. 60 µL of effector cell suspension mix was pipetted into the inlet and 60µl was withdrawn from the outlet, to obtain a homogenous distribution of effector cells along the length of the channel. The slide was incubated upside down for 5 minutes in a humidity and CO₂ controlled incubator. Three images were taken using the fluorescent microscope, one close to the inlet, one in the center of the channel and one close to the outlet. Each image had Brightfield, FarRed, Violet and Green channels. The slide was put back in the centrifuge and spun at 250xg for 2 minutes applying a force to the effector cells in a direction away from the target cell layer (as indicated in Figure 5. The slide was taken out and again three fluorescent images were taken in the same channel positions. The previous step was repeated, increasing the speed to 500xg, 1000xg, 2000xg, 4000xg.

### Shear-flow experiments

z-Movi^{®} chips were used for these experiments (without the use of acoustic force actuation). Monolayer seeding was performed in the same way as for the acoustic force experiments. The chip was placed and secured on the Nikon Ti2 microscope using a z-Movi^{®} chip holder. A tube to the pump syringe was connected to the valve outlet of the chip, with chip valve closed. The valve was then opened and a ramp of 0 - 1000 µl/min over a period of 2:30 minutes was performed with a programmable Harvard Apparatus syringe pump. This corresponded to maximum shear stress of around 20 Pa in the flow channel. This ramp was done first to check the monolayer stability (and to remove any cells that were not well attached to the surface). After that, the stained effector cells were mixed in a 1:1:1 ratio and introduced into the flow channel. The chip was incubated for 5 minutes at room temperature. A flow ramp of 0 - 1000 µl/min within 2:30 min was applied with the syringe pump while images were taken in Brightfield, FarRed, Violet channel and Green channels every 20 msec using the microscope. Data was analyzed using NIS Elements software (Nikon).

### Summary and interpretation of results

The cellular avidity curves and percentage of bound cells determined for the different experimental conditions (acoustic force, centrifugation and shear force) comparing the cells having different expression levels (UNT, CAR Low, CAR High) indicate that the cellular avidity curve and percentage bound cells is affected when cells have different receptor expression levels (See Figures 6-8). Inventors have found that not only the absence or presence of a receptor and the type of receptor play a role in determining the avidity of a cell with respect to another cell, but that also the expression level plays an important role.

Furthermore, the cellular avidity curves show that substantial enrichment can be obtained when utilizing cellular avidity as a means to separate high and low expressing cells. In particular Figure 8 shows that a single round of shear force separation allows to have 97.6% of low expressing cells unbound, while 45% of high expressing cells remain attached. if one would start with a mixture of 1000 CAR High and 1000 CAR low cells (50%, 50%) one would end up with around 450 CAR High cells and 24 CAR low cells in the bound fraction. If one would recover those one would end up with (CAR High: 95%, CAR Low: 5%). For the flow through fraction, one would end up with 550 CAR High cells and 976 Low CAR cells (CAR High: 36%, CAR Low: 64%).

It can be calculated that carrying out this step 3 times consecutively, each time collecting the flow through fraction and using that for a next enrichment run, would result in a yield of about 93% of the low CAR expressing cells while having a purity of about 85% (see Table 1 for details).

Similarly, In Figure 7 the relatively flat curves for the CAR High and the UNT at forces above 250xg indicate that if one would start with a sample of 100 CAR High cells, 100 CAR Low cells and 100 UNT cells (33%, 33%, 33%) one would end up after spinning 2 minutes at 250xg with (82, 63, 30) cells in the bound fraction. Then moving the slide over to a clean centrifuge tube and increasing the force to 2000xg one would end up with (in this case) 63-37 = 26 CAR Low cells in the unbound fraction together with 82-76 = 6 CAR High cells and 30-24 = 6 UNT cells leading to a ratio of (16%, 68%, 16%) for (CAR High, CAR Low, UNT) in the recovered unbound fraction.

To conclude, this means based on the data show here that cellular avidity can be used as a unit of measure which correlates with expression levels. This may provide for a meaningful parameter, as expression level may be an important parameter that may allow one to control/regulate cellular avidity, which can be of important clinical relevance. For example, a cell with a certain expression level of a receptor may be less effective in certain test conditions and/or functional or clinical tests. By tailoring expression levels and measuring the cellular avidity of the modified cells with respect to certain target cells the avidity and the effectiveness of the cells carrying the receptor may be optimized.

Moreover, the data also shows that cells may be enriched for subpopulations having different expression levels, for example, as a result of different copy numbers and/or different insertion sites (having different transcription rates). Hence, in case it is desirable to enrich certain fractions, cellular avidity enrichment can be used as a relatively mild process step in the preparation of cell products, e.g. cell therapy products or the like.

### Example 2

### Sorting of a heterogeneous population of effector cells having different expression levels by means of cellular avidity

Effector cells (*i.e.* primary human T cells) were purified from buffy coats using Ficoll density gradient centrifugation and subsequent magnetic bead selection using methods generally known in the art.

Next, the effector cells were subjected to gamma retroviral transduction to transduce them with a CAR-FMC63 anti-CD19 construct (Kramer, AM; (2017) Delineating the impact of binding-domain affinity and kinetic properties on Chimeric Antigen Receptor T-cell function. Doctoral thesis, UCL (University College London)). Next, the transduction efficiency and construct expression were measured by means of median fluorescence intensity (MFI) using an antibody specific for a marker of the transfected construct and using methods generally known in the art.

Thereafter, the effector cells were stained with CellTrace^{™} Violet (Invitrogen) for 15 minutes at 37°C and then resuspended in complete RPMI medium. A fraction of 100.000 cells were taken aside, placed in a 1.5 ml tube and kept at 37°C in an incubator. This fraction was called the INPUT.

NALM6 (CD19+) cells (from ATCC) were used as target cells. A monolayer of target cells was made in a channel slide as shown in Figure 3 having a channel height of 200 µm. The monolayer of target cells was attached to the ceiling of the channel using the process as described herein and as schematically shown in Figure 5.

Target cells were counted and suspended in serum-free RPMI medium at a concentration (80e6 cells/ml) required for a confluence close to 100%. Sixty µl of the cell suspension was pipetted into the inlet and introduced in the channel by tilting the slide for a few seconds until the suspension reached the outlet. The volume of the channel is 60 µl, so the whole channel was filled. The liquid in the channel was kept from moving by means of the surface tension at the level of the inlets, as only the channel was filled with liquid while the inlets were kept empty.

The slide was flipped straight away and incubated upside down in a humidity and CO₂ controlled incubator for 30 minutes at 37°C. The slide was put upright again and medium was exchanged by adding 100 µl of serum containing RPMI medium into the inlet and withdrawing 100 µl from the outlet. This procedure was repeated four times. The slide was put upside down again and incubated in the humidity and CO₂ controlled incubator for an additional 30 minutes at 37°C.

Next, the slide was placed into an adaptor (see Figure 4) of a centrifuge bucket and the cells were subjected to a force by spinning for 2 minutes at 1000xg to remove target cells that were not attached well to the channel ceiling.

The slide was taken out of the centrifuge and 60 µL of the mixture of effector cells was pipetted into the inlet and 60 µL was withdrawn from the outlet to obtain an homogenous distribution of effector cells within the channel. The slide was flipped again and incubated for 5 minutes in the humidity and CO2 controlled incubator at 37°C.

Next, images were acquired using the fluorescent microscope in three channel positions, one close to the inlet, one in the center of the channel and one close to the outlet. Each image had a Brightfield + Violet channel.

The slide was placed back in the centrifuge and the cells were subjected to a force in a direction away from the target cell monolayer by spinning at 1000xg for 2 minutes.

The slide was taken out of the centrifuge again and images were acquired using the fluorescent microscope in the same three channel positions.

After centrifugation, the medium present in the inlets was removed and placed in an empty 15 ml tube (only the amount of medium to empty the inlets as such was removed, the channel was still completely filled with medium). Medium was then exchanged by applying 100 µl of serum-containing medium into the inlet and withdrawing 100 µl from the outlet. This procedure was repeated four times. All the medium removed from the channel was placed in the 15 ml tube. This fraction was called the OUTPUT.

Next, the following flow cytometer staining mix was prepared. Viability staining eFluor^{™} 780 (Thermofisher) was combined with the antibody specific for a marker of the transfected construct as used in the MFI measurement as described above.

The Input and Output tubes were spun down for 5 minutes at 400xg to obtain two separate cell pellets. The medium was removed and each of the pellets were resuspended in 200 µl of the flow cytometer staining mix. The resuspended pellets were incubated for 60 minutes at room temperature in the dark, then spun down for 5 minutes at 400xg to obtain two separate cell pellets. The pellets were resuspended in 200 µl of PBS and introduced into a flow cytometer for reading. CellTrace^{™} Violet was detected using a 405 nm laser and a 450/45 BF filter (PB450 channel), while the viability dye was detected using a 638 nm laser and a 780/60 BF filter (APC-750 channel). The construct marker was detected (using MFI) by using a 561 nm laser and a 585/42 BF filter (PE channel) After applying the cells/debris gate, viable cells gate and CellTrace^{™} Violet gate (to remove monolayer cells from the Output count), the MFI for the transfected construct was measured (see Table 2).

The data show that the MFI of the OUTPUT fraction was reduced significantly compared to the MFI of the INPUT fraction. Based on this it is clear that the effector cells that remained attached to the target cells after force application have a higher MFI compared to the INPUT fraction. As MFI is a measure of CAR expression, the effector cells that remained attached to the target cells are enriched in high CAR expressing cells.

**Table 1. Number of cells recovered for three consecutive runs through a shear-flow sorting chip.**

| | Number of cells | | | |
|---|---|---|---|---|
| | Start | Run 1 | Run 2 | Run 3 |
| CAR High | 1000 | 550 | 303 | 166 |
| CAR Low | 1000 | 976 | 953 | 930 |

**Table 2: MFI value of INPUT and OUTPUT fractions.**

| Fraction | MFI |
|---|---|
| INPUT | 89491 |
| OUTPUT | 17317 |

## Claims

1. A method for differentiating between cells having different expression levels of a receptor by determining cellular avidity, comprising the steps of:
a) providing cells (6) with different expression levels of a receptor:
b) providing target cells (2) attached to a surface (1);
c) contacting each of the cells with different expression levels of the receptor with the target cells attached to a surface to allow the cells to interact with the target cells;
d) exerting a force (7) on the cells with different expression levels of the receptor, wherein the force is in a direction away from the target cells;
e) determining of each of the cells with different expression levels of the receptor the number of cells that detached from and/or remained attached to the target cells;
f) assigning cellular avidity scores to each of the cells with different expression levels.

2. Method in accordance with claim 1, wherein each of the cells with different expression levels is provided with a different label, and the contacting step is performed with the different cells combined.

3. Method in accordance with claim 1, wherein each of the cells with different expression levels is provided separately, and separate contacting steps are performed.

4. Method in accordance with any one of claims 1 to 3, wherein the cells with different expression levels of the receptor are provided by providing cells with different expression constructs expressing the same receptor and/or providing cells with different copy numbers of an expression construct expressing the same receptor.

5. The method in accordance with any one of claims 1 to 4, wherein for each cell with a different expression level, the percentage of the total number of cells that remained bound relative to the cells that were contacted with target cell attached to the surface is determined as a cellular avidity score.

6. The method in accordance with any one of claims 1 to 5, further comprising ranking each of the cells with different expression levels based on avidity scores.

7. Method in accordance with any one of claims 1 to 6, wherein the cells having different expression levels of a receptor are effector cells.

8. Method in accordance with claim 7, wherein in effector cells bound with target cells after the step of applying the force the presence of a marker associated with synapse formation is determined.

9. Method in accordance with claim 8, wherein cellular avidity scores are determined based on the number of effector cells with the marker associated with synapse formation.

10. A method for enriching a particular cell population, comprised in a population of cells comprising cells with different expression levels of a receptor, said particular cell population having a relatively higher or lower expression level of the receptor:
- providing target cells (2) attached to a surface (1);
- contacting a population of cells (6) with different expression levels of a receptor with the target cells attached to the surface to allow the population of cells to interact with the target cells;
- exerting a force (7) on the population of cells with different expression levels of the receptor, wherein the force is in a direction away from the target cells;
- collecting cells from the cell population which detached from and/or remained attached to the target cells attached to the surface, therewith providing fractions comprising enriched cell populations comprising a relatively higher or lower expression level of the receptor.

11. A method for enriching a cell population comprising a range of copy numbers of a gene construct expressing a receptor, said method comprising:
- providing target cells (2) attached to a surface (1);
- providing a cell population (6) comprising different copy numbers of the gene construct expressing the receptor; said copy numbers ranging from 1 to 2, or more;
- contacting the cell population with the target cells attached to a surface;
- exerting a force (7) on the cell population, wherein the force is in a direction away from the target cells;
- collecting cells from the cell population which detached from and/or remained attached to the target cells attached to the surface, therewith providing fractions comprising enriched cell populations comprising a relatively higher or lower copy number of the gene construct.

12. The method in accordance with claim 11, wherein the lower copy number is 1.

13. The method in accordance with any one of claims 1 to 12, wherein the applied force is an acoustic force, a shear flow force or a centrifugal force.

14. The method in accordance with any one of claims 1 to 13, wherein the target cell presents a cancer antigen, or wherein the target cells are cancer cells.

15. Method in accordance with any one of claims 1 to 14, wherein an agent capable of modulating the interaction between the cells with a receptor and the target cell is included in the contacting and force exerting steps.

## Patentansprüche

1. Verfahren zur Unterscheidung zwischen Zellen mit unterschiedlichen Expressionsniveaus eines Rezeptors durch Bestimmen der Zellavidität, umfassend die Schritte:
a) Bereitstellen von Zellen (6) mit unterschiedlichen Expressionsniveaus eines Rezeptors:
b) Bereitstellen von Zielzellen (2), die an eine Oberfläche (1) gebunden sind;
c) In-Kontakt-Bringen jeder der Zellen mit unterschiedlichen Expressionsniveaus des Rezeptors mit den an eine Oberfläche gebundenen Zielzellen, um den Zellen die Wechselwirkung mit den Zielzellen zu ermöglichen;
d) Ausüben einer Kraft (7) auf die Zellen mit unterschiedlichen Expressionsniveaus des Rezeptors, wobei die Kraft in einer Richtung weg von den Zielzellen verläuft;
e) Bestimmen bei jeder der Zellen mit unterschiedlichen Expressionsniveaus des Rezeptors die Zahl der Zellen, die sich von den Zielzellen ablösten und/oder an diesen gebunden blieben;
f) Zuweisen von Zellaviditätswertungen an jede der Zellen mit unterschiedlichen Expressionsniveaus.

2. Verfahren gemäß Anspruch 1, wobei jede der Zellen mit unterschiedlichen Expressionsniveaus mit einer anderen Markierung versehen ist und der Kontaktierungsschritt mit den unterschiedlichen Zellen kombiniert durchgeführt wird.

3. Verfahren gemäß Anspruch 1, wobei jede der Zellen mit unterschiedlichen Expressionsniveaus separat bereitgestellt wird und separate Kontaktierungsschritte durchgeführt werden.

4. Verfahren gemäß einem der Ansprüche 1 bis 3, wobei die Zellen mit unterschiedlichen Expressionsniveaus des Rezeptors bereitgestellt werden, indem Zellen mit unterschiedlichen Expressionskonstrukten, die den gleichen Rezeptor exprimieren, und/oder Zellen mit unterschiedlichen Kopienzahlen eines Expressionskonstrukts, das den gleichen Rezeptor exprimiert, bereitgestellt werden.

5. Verfahren gemäß einem der Ansprüche 1 bis 4, wobei für jede Zelle mit einem unterschiedlichen Expressionsniveau der Prozentsatz der Gesamtzahl der Zellen, die gebunden blieben, bezüglich der Zellen, die mit einer an die Oberfläche gebundenen Zielzelle in Kontakt gebracht wurden, als Zellaviditätswertung bestimmt wird.

6. Verfahren gemäß einem der Ansprüche 1 bis 5, ferner umfassend Erstellen einer Rangliste jeder der Zellen mit unterschiedlichen Expressionsniveaus bezogen auf Aviditätswertungen.

7. Verfahren gemäß einem der Ansprüche 1 bis 6, wobei es sich bei den Zellen mit unterschiedlichen Expressionsniveaus eines Rezeptors um Effektorzellen handelt.

8. Verfahren gemäß Anspruch 7, wobei in Effektorzellen, die nach dem Schritt des Anlegens der Kraft mit Zielzellen gebunden sind, das Vorliegen eines mit Synapsenbildung assoziierten Markers bestimmt wird.

9. Verfahren gemäß Anspruch 8, wobei Zellaviditätswertungen aufgrund der Zahl der Effektorzellen mit dem mit Synapsenbildung assoziierten Marker bestimmt werden.

10. Verfahren zum Anreichern einer bestimmten Zellpopulation, die in einer Population von Zellen enthalten ist, die Zellen mit unterschiedlichen Expressionsniveaus eines Rezeptors umfasst, wobei die bestimmte Zellpopulation ein relativ höheres oder niedrigeres Expressionsniveau des Rezeptors aufweist:
- Bereitstellen von Zielzellen (2), die an eine Oberfläche (1) gebunden sind;
- In-Kontakt-Bringen einer Population von Zellen (6) mit unterschiedlichen Expressionsniveaus eines Rezeptors mit den an die Oberfläche gebundenen Zielzellen, um der Population von Zellen die Wechselwirkung mit den Zielzellen zu ermöglichen;
- Ausüben einer Kraft (7) auf die Population von Zellen mit unterschiedlichen Expressionsniveaus des Rezeptors, wobei die Kraft in einer Richtung weg von den Zielzellen verläuft;
- Sammeln von Zellen aus der Zellpopulation, die sich von den an die Oberfläche gebundenen Zielzellen ablösten und/oder an diesen gebunden blieben, wodurch Fraktionen bereitgestellt werden, die angereicherte Zellpopulationen umfassen, die ein relativ höheres oder niedrigeres Expressionsniveau des Rezeptors umfassen.

11. Verfahren zum Anreichern einer Zellpopulation, die ein Spektrum von Kopienzahlen eines einen Rezeptor exprimierenden Genkonstrukts umfasst, wobei das Verfahren Folgendes umfasst:
- Bereitstellen von Zielzellen (2), die an eine Oberfläche (1) gebunden sind;
- Bereitstellen einer Zellpopulation (6), die unterschiedliche Kopienzahlen des den Rezeptor exprimierenden Genkonstrukts umfasst; wobei die Kopienzahlen im Bereich von 1 bis 2 oder mehr liegen;
- In-Kontakt-Bringen der Zellpopulation mit den an eine Oberfläche gebundenen Zielzellen;
- Ausüben einer Kraft (7) auf die Zellpopulation, wobei die Kraft in einer Richtung weg von den Zielzellen verläuft;
- Sammeln von Zellen aus der Zellpopulation, die sich von den an die Oberfläche gebundenen Zielzellen ablösten und/oder an diesen gebunden blieben, wodurch Fraktionen bereitgestellt werden, die angereicherte Zellpopulationen umfassen, die eine relativ höhere oder niedrigere Kopienzahl des Genkonstrukts umfassen.

12. Verfahren gemäß Anspruch 11, wobei die niedrigere Kopienzahl 1 ist.

13. Verfahren gemäß einem der Ansprüche 1 bis 12, wobei es sich bei der angelegten Kraft um eine akustische Kraft, eine Scherströmungskraft oder eine Zentrifugalkraft handelt.

14. Verfahren gemäß einem der Ansprüche 1 bis 13, wobei die Zielzelle ein Krebsantigen präsentiert oder wobei es sich bei den Zielzellen um Krebszellen handelt.

15. Verfahren gemäß einem der Ansprüche 1 bis 14, wobei ein Agens, das in der Lage ist, die Wechselwirkung zwischen den Zellen mit einem Rezeptor und der Zielzelle zu modulieren, in den Kontaktierungs- und Kraftausübungsschritten enthalten ist.

## Revendications

1. Procédé de différenciation entre des cellules ayant différents niveaux d'expression d'un récepteur en déterminant l'avidité cellulaire, comprenant les étapes :
a) de fourniture de cellules (6) ayant différents niveaux d'expression d'un récepteur ;
b) de fourniture de cellules cibles (2) attachées à une surface (1) ;
c) de mise en contact de chacune des cellules ayant différents niveaux d'expression du récepteur avec les cellules cibles attachées à une surface afin de permettre aux cellules d'interagir avec les cellules cibles ;
d) d'application d'une force (7) sur les cellules ayant différents niveaux d'expression du récepteur
dans lequel la force est dans une direction d'écartement par rapport aux cellules cibles ;
e) de détermination, pour chacune des cellules ayant différents niveaux d'expression du récepteur, du nombre de cellules qui se sont détachées de et/ou sont restées attachées aux cellules cibles ;
f) d'attribution de scores d'avidité cellulaire à chacune des cellules ayant différents niveaux d'expression.

2. Procédé selon la revendication 1, dans lequel chacune des cellules ayant différents niveaux d'expression est munie d'une étiquette différente, et l'étape de mise en contact est réalisée avec les différentes cellules combinées.

3. Procédé selon la revendication 1, dans lequel chacune des cellules ayant différents niveaux d'expression est fournie séparément, et des étapes de mise en contact séparées sont réalisées.

4. Procédé selon l'une quelconque des revendications 1 à 3, dans lequel les cellules ayant différents niveaux d'expression du récepteur sont fournies en fournissant des cellules ayant différentes constructions d'expression exprimant le même récepteur et/ou en fournissant des cellules ayant différents nombres de copies d'une construction d'expression exprimant le même récepteur.

5. Procédé selon l'une quelconque des revendications 1 à 4, dans lequel, pour chaque cellule ayant un niveau d'expression différent, le pourcentage du nombre total de cellules qui sont restées liées par rapport aux cellules qui ont été mises en contact avec la cellule cible attachée à la surface est déterminé comme un score d'avidité cellulaire.

6. Procédé selon l'une quelconque des revendications 1 à 5, comprenant en outre le classement de chacune des cellules ayant différents niveaux d'expression sur la base des scores d'avidité.

7. Procédé selon l'une quelconque des revendications 1 à 6, dans lequel les cellules ayant différents niveaux d'expression d'un récepteur sont des cellules effectrices.

8. Procédé selon la revendication 7, dans lequel, dans les cellules effectrices liées aux cellules cibles après l'étape d'application de la force, la présence d'un marqueur associé à la formation de synapse est déterminée.

9. Procédé selon la revendication 8, dans lequel les scores d'avidité cellulaire sont déterminés sur la base du nombre de cellules effectrices avec le marqueur associé à la formation de synapse.

10. Procédé d'enrichissement d'une population cellulaire particulière, comprise dans une population de cellules comprenant des cellules ayant différents niveaux d'expression d'un récepteur, ladite population cellulaire particulière ayant un niveau d'expression relativement plus élevé ou plus faible du récepteur :
- de fourniture de cellules cibles (2) attachées à une surface (1) ;
- de mise en contact d'une population de cellules (6) ayant différents niveaux d'expression d'un récepteur avec les cellules cibles attachées à la surface afin de permettre à la population de cellules d'interagir avec les cellules cibles ;
- d'application d'une force (7) sur la population de cellules ayant différents niveaux d'expression du récepteur, où la force est dans une direction d'écartement par rapport aux cellules cibles ;
- de collecte de cellules de la population cellulaire qui se sont détachées de et/ou sont restées attachées aux cellules cibles attachées à la surface, fournissant ainsi des fractions comprenant des populations cellulaires enrichies comprenant un niveau d'expression relativement plus élevé ou plus faible du récepteur.

11. Procédé d'enrichissement d'une population cellulaire comprenant une plage de nombres de copies d'une construction génique exprimant un récepteur, ledit procédé comprenant :
- la fourniture de cellules cibles (2) attachées à une surface (1) ;
- la fourniture d'une population cellulaire (6) comprenant différents nombres de copies de la construction génique exprimant le récepteur ; lesdits nombres de copies se trouvant dans la plage comprise entre 1 et 2, ou plus ;
- la mise en contact de la population cellulaire avec les cellules cibles attachées à une surface ;
- l'application d'une force (7) sur la population cellulaire, où la force est dans une direction loin des cellules cibles ;
- la collecte de cellules de la population cellulaire qui se sont détachées de et/ou sont restées attachées aux cellules cibles attachées à la surface, fournissant ainsi des fractions comprenant des populations cellulaires enrichies comprenant un nombre de copies relativement plus élevé ou plus faible de la construction génique.

12. Procédé selon la revendication 11, dans lequel le nombre de copies plus faible est 1.

13. Procédé selon l'une quelconque des revendications 1 à 12, dans lequel la force appliquée est une force acoustique, une force de flux de cisaillement ou une force centrifuge.

14. Procédé selon l'une quelconque des revendications 1 à 13, dans lequel la cellule cible présente un antigène du cancer, ou dans lequel les cellules cibles sont des cellules cancéreuses.

15. Procédé selon l'une quelconque des revendications 1 à 14, dans lequel un agent capable de moduler l'interaction entre les cellules avec un récepteur et la cellule cible est inclus dans les étapes de mise en contact et d'application de force.
